# EUROPEAN PATENT SPECIFICATION

(11) **EP 3 708 533 B1**
(45) Date of publication and mention of the grant of the patent: **07.06.2023**
(21) Application number: 19163136.5
(22) Date of filing: 15.03.2019
(51) Int. Cl.: B82Y 35/00, B82Y 40/00, G01R 33/12, G01R 33/022, A61B 5/05

(54) **METHOD AND APPARATUS FOR IN-SYNTHESIS SENSING AND CONTROL OF MAGNETIC NANOSTRUCTURES**
VERFAHREN UND VORRICHTUNG ZUM ERFASSEN UND STEUERN VON MAGNETISCHEN NANOSTRUKTUREN IN DER SYNTHESE
PROCÉDÉ ET APPAREIL DE DETECTION ET DE CONTRÔLE EN SYNTHÈSE DE NANOSTRUCTURES MAGNÉTIQUES

(43) Date of publication of application: 16.09.2020
(73) Proprietor: Universität zu Lübeck, 23562 Lübeck (DE)
(72) Inventor: Malhotra, Ankit, 23562 Lübeck (DE); von Gladiß, Anselm, 56072 Koblenz (DE); Lüdtke-Buzug, Kerstin, 23562 Lübeck (DE); Buzug, Thorsten, 23627 Groß Sarau (DE)
(74) Representative: Wallinger, Michael

(56) References cited:
- WO-A1-2017/062821
- DE-A1-102016 116 264
- US-A1- 2015 008 910
- US-A1- 2016 158 387

## Description

### Field of invention

The invention relates to the synthesis of tailored magnetic nanoparticles and methods for using these nanoparticles for solving biological problems such as diagnosis and therapy. Magnetic nanostructures in the form of particles, rods, chains, aggregates etc. have immense technical, biological and medical applications. In engineering, magnetic nanostructures are used e.g. for data-storage applications. Medical applications include medical diagnostics (e.g. magnetic particle imaging (MPI) and magnetic resonance imaging (MRI)), drug targeting and innovative cancer therapies such as magnetic fluid hyperthermia (MFH).

### Description of prior art

Synthesis and fabrication of nanostructures are ongoing scientific topics for the last few decades. Different nanostructures have been synthesized for various applications but the focus to characterize these nanostructures in terms of magnetic (e.g. saturation magnetization, steepness of magnetization curve, anisotropy etc.) and physical properties (such as shape, size, size distribution etc.) has become more important with the usage in the field of biology and medical science. A combination of these nanostructures with the existing biological research instrumentation has provided a powerful tool in clinical and pre-clinical environment for diagnosis and therapy. One of the latest example from the last decade is MPI which is a novel imaging modality measuring the spatial distribution of superparamagnetic iron oxide nanoparticles.

From DE 10 2017 108 737 A1, a method and a device for producing dispersions of magnetic nanoparticles are known, which nanoparticles are suitable for both MPI and MFH. The physical properties of the nanoparticles are steered by using sound waves in the form of ultrasonic waves and by using static and dynamic magnetic fields.

Moreover, CN 105268385 A discloses a reaction device for preparing a magnetic nanomaterial through ultrasound enhancement cooperating with magnetic field induction.

WO 2017/062821 A1 discloses a system configured to characterize a magnetic response of a sample. The system can comprise an electrical source configured to generate a time-varying current supply, an excitation coil system coupled to the electrical source to generate a time-varying magnetic field for application to a sample, and a sensing coil system that senses a magnetic response of the sample in response to the time-varying magnetic field.

DE 10 2016 116 264 A1 discloses a magnetic-field imager including a plurality of coils (e.g., an array of coils deployed through the magnetic-field imager) for detecting changes in magnetic fields. Each coil can define a pixel within the magnetic field image sensor. In this manner, the array of coils defines an active sensor area where a fluid sample including cells, viruses, and other entities can be deposited over such that respective coils can detect a change in the magnetic field caused by magnetic nanoparticles or superparamagnetic nanoparticles.

US 2015/0008910 A1 discloses a magnetic particle imaging apparatus including magnets that produce a gradient magnetic field having a field free region, excitation field electromagnets that produce a radiofrequency magnetic field within the field free region, and high-Q receiving coils that detect a response of magnetic particles in the field free region to the excitation field. Intermodulated low and radio-frequency excitation signals are processed to produce an image of a distribution of the magnetic nanoparticles within the imaging region.

US 2016/0158387 A1 discloses a method for selecting a particular magnetic nanoparticle size that is optimized for diagnostic and/or therapeutic applications, for example, a method of producing magnetic nanoparticles with a tuned size distribution for magnetic particle imaging.

Apart from these synthesis techniques, there are various techniques or devices available for characterizing the nanoparticles such as vibrating sample magnetometers (measuring the static magnetization), AC magnetometers (measuring the magnetic susceptibility), and magnetic particle spectrometers such as arbitrary-waveform spectrometers. However, these devices can only characterize the nanoparticles after the synthesis process. Hence, the complete synthesis process is unobserved and no information regarding the nucleation and growth of the nanostructures is measured. A fast feedback for manipulating the synthesis process is currently not available.

For manipulating the growth of the nanostructures during their synthesis, the most essential tools are
1) a sensing device, which measures the changes in the properties of the nanostructures in real time and
2) a feedback loop, which steers the nucleation and growth process for improving
   the physical properties, especially the magnetic properties of the nanostructures. There are a handful of devices, which are capable for real time monitoring of nanostructures, and based on techniques such as x-ray scattering and transmission electron microscopy (TEM), but these devices require the most sophisticated engineering and special conditions for monitoring and analysis. Hence, it comes with great effort using these devices in a normal chemistry environment.

Thus, the problem underlying the present invention is to provide a sensing device for magnetic nanostructures, a feedback loop for steering the nucleation and growth process of the magnetic nanostructures, and corresponding methods which are fast and simple. This problem is solved by the method for sensing magnetic nanostructures, the method for sensing the growth of magnetic nanostructures, the method for steering the growth of magnetic nanostructures, the method for obtaining magnetic nanostructures, and, correspondingly, the device for sensing magnetic nanostructures, the device for steering the growth of magnetic nanostructures, and the device for obtaining magnetic nanostructures according to the independent claims.

Further advantageous embodiments of the invention are contained in the dependent subclaims.

### Summary of the invention

A first aspect of the present invention relates to a method for steering the growth of magnetic nanostructures according to claim 1.

In a preferred embodiment of the first aspect, the growth of the magnetic nanostructures is influenced by applying an external field, in particular a magnetic, electrical, electro-magnetic, and/or ultrasonic field, to the magnetic nanostructures.

Due to the application of the external field, magnetic properties of the magnetic nanostructures such as the amplitude, the phase, or the hysteresis of the magnetic nanostructures, can be changed.

Due to the application of the external field, also physical properties of the magnetic nanostructures such as the shape or the size distribution of the particles can be changed, and/or the formation of clusters, tubes, or rod like structures can be induced.

A second aspect of the present invention relates to a method for obtaining magnetic nanostructures according to claim 8.

A third aspect of the present invention relates to a device for steering the growth of magnetic nanostructures according to claim 9.

A fourth aspect of the present invention relates to a device for obtaining magnetic nanostructures according to claim 10.

Further advantageous embodiments of the aspects previously specified are described in the following description in connection with the attached drawings and tables and in the dependent claims. It is shown in:
- Fig. 1A:: a block diagram of a sensing device for sensing magnetic nanostructures;
- Fig. 1B:: a block diagram of another variant of the sensing device according to Fig. 1A;
- Fig. 2:: a device according to the invention for steering the growth of nanostructures with the help of external magnetic fields;
- Fig. 3:: a device according to the invention for steering the growth of nanostructures with chemical/biological reagents;
- Fig. 4:: a cross-section through the housing of a sensing device for sensing magnetic nanostructures;
- Fig. 5:: a nucleation and growth curve acquired during the synthesis of nanostructures;
- Fig. 6:: the measurements of the amplitude and phase spectra for the points marked by large dots in Fig. 5;
- Fig. A:: the spectral magnetic moments and the hysteresis curves of all the synthesized samples at different field strengths measured with 1D MPS;
- Fig. B:: the magnetic moment of the third harmonics versus time for the entire synthesis process for Sample 16 and Sample 17;
- Fig. C:: the final amplitude spectra and the magnetization curve for Sample 16 and Sample 17 at the end of the synthesis process;
- Fig. D:: the magnetic moment of the third harmonics versus time for the entire synthesis process for Sample 18 and Sample 19;
- Fig. E:: the final amplitude spectra and the magnetization curve for Sample 16 and Sample 17 at the end of the synthesis process;
- Fig. F:: the magnetic moment of the third harmonics versus time for the entire synthesis process for Sample 20 and Sample 21;
- Fig. G:: the final amplitude spectra and the magnetization curve for Sample 20 and Sample 21 at the end of the synthesis process;
- Fig. H:: the magnetic moment of the third harmonics versus time for the entire synthesis process for Sample 22 to Sample 24;
- Fig. I:: the final amplitude spectra and the magnetization curve for Sample 22 to Sample 24 at the end of the synthesis process;
- Fig. J:: the magnetic moment of the third harmonics versus time for the entire synthesis process for Sample 25 and Sample 26;
- Fig. K:: the final amplitude spectra and the magnetization curve for Sample 25 and Sample 26 at the end of the synthesis process;
- Fig. L:: the final amplitude spectra for Sample 4 to Sample 6 at the end of the synthesis process measured in 1D MPS;
- Fig. M:: the magnetization curve for Sample 1 and Sample 2 at the end of the synthesis process measured with 1D MPS;

- Fig. N:: the final amplitude spectra and the magnetization curve for Sample 1 and Sample 2 at the end of the synthesis process measured with 1D MPS;
- Fig. O:: the magnetization curves of all the synthesized samples (Sample 8 and Sample 9) at different field strengths measured with 1D MPS; moreover, a comparison with commercially available nanoparticles;
- Fig. P:: an exemplary algorithm to control the nucleation and growth of nanostructures in a continuous cycle;

- Table A:: parameters for the synthesis of nanostructures in static magnetic fields (Experiment I);
- Table B:: parameters for the synthesis of nanostructures with different flow rates (Experiment II);
- Table C:: parameters for the synthesis of nanostructures with different flow rates (Experiment II)
- Table D:: parameters for the synthesis of nanostructures with different quantities of dextran (Experiment III)
- Table E:: parameters for the synthesis of nanostructures with different quantities of dextran (Experiment III);
- Table F:: parameters for the synthesis of nanostructures without dextran (Experiment IV);
- Table G:: parameters for the synthesis of nanostructures (Experiment V);
- Table H:: parameters for the synthesis of nanostructures in homogenous magnetic AC fields (Experiment VI);
- Table I:: parameters for the synthesis of nanostructures in static magnetic fields;
- Table J:: the effect of key parameters on the properties of nanostructures.

### Sensing apparatus

The methods and devices according to the first to fourth aspects specified above use a consolidation of physics, chemistry and engineering.

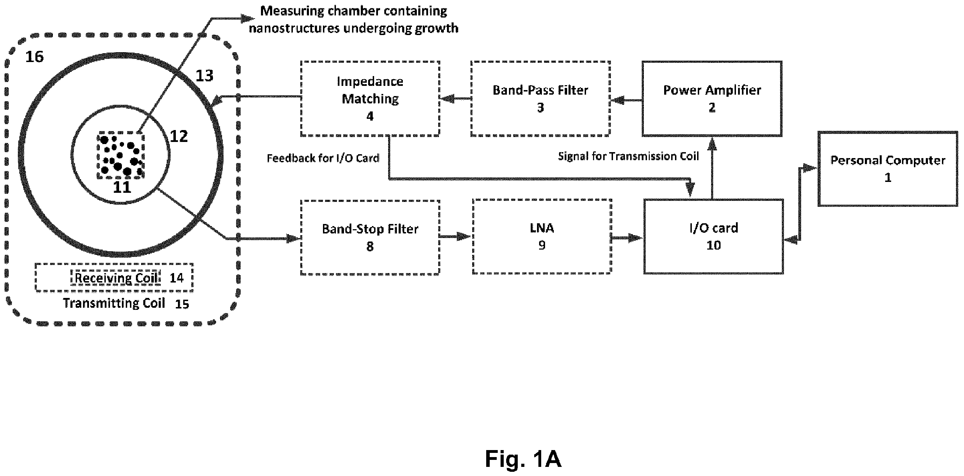

Fig. 1A shows a block diagram of a sensing apparatus for sensing magnetic nanostructures . The sensing apparatus comprises a transmitting coil 13 for exciting the nanostructures and a receiving coil 12 (in this embodiment a gradiometer coil) for acquiring the signals from the nanostructures. A power amplifier 2 produces the required excitation field, followed by a band-pass filter 3 for reducing the attenuations produced by the power amplifier. The impedance matching 4 optimizes the maximum power transmission between the power amplifier and transmitting coil. At the receive channel the device may comprise of a band-stop filter 8 for attenuating the excitation frequency getting coupled with the receiving coil 12, followed by a low noise amplifier 9 for amplifying the signals obtained from nanostructures under synthesis. The I/O card 10 and personal computer 1 are used for controlling the device as well as for displaying the acquired data. The blocks without the dashed lines are the ones realized for a current lab-prototype apparatus.

In more detail, the device according to Fig. 1A comprises a computing device or personal computer 1 for displaying the data and setting the operational parameters for the device. An I/O card 10, which includes analogue to digital convertors (ADC) and digital to analogue convertors (DAC), generates the excitation signal for the transmitting coil 13 and digitizes the voltage received by the receiving coil 12. The nanostructures are placed in the center of the apparatus. The housing 16 consists of the mechanical structures for holding the synthesis flask 11, the transmitting coil 13 and the receiving coil 12. Moreover, the cooling of the transmitting coil 13 and the receiving coil 12 is performed via channels present in the housing by passing pressurized air.

To produce a field for excitation of the nanostructures, a power amplifier amplifies the excitation signal generated by the I/O card 10. The device according to Fig. 1A uses an excitation frequency (Tₓ) of 23 kHz and a magnetic field of 10 mT. However, the parameters may be changed. A bandpass filter 3 can be used for filtering the disturbances produced by the power amplifier. For transmitting the maximum power to the transmitting coil 13, an impedance matching unit 4 is used that consists of a series resistor and parallel resistor. The receiving coil 12 is located inside the transmitting coil 13 leading to higher mutual inductance causing a direct feed through of the excitation signal to the receiving coil 12.

The transmitting signal is of 5-10 higher order in comparison to the signals from the nanostructures. Therefore, it is important to attenuate the transmitting signal. There are a number of techniques to attenuate the excitation frequency such as using band stop filters 8, parallel sensing, gradiometer coils or a combination of these techniques. As the amplitude of the signal received from the nanostructures could be in the order of 10⁻⁹ to 10⁻⁶ V, it is essential to amplify the signal to the dynamic range of the I/O card 10 with a low noise amplifier (LNA) 9 before recording the data. In the device according to Fig. 1A, a gradiometer coil (as a receiving coil 12) achieves attenuation of the excitation frequency. A careful design with the gradiometer coil can eliminate the need of a band-stop filter 8 as well as the LNA 9.

Moreover, it is also possible to use a single coil for transmitting the excitation signal as well as for receiving the nanostructure response.

A brief summary of the technical details of the device according to Fig. 1A is given further below.

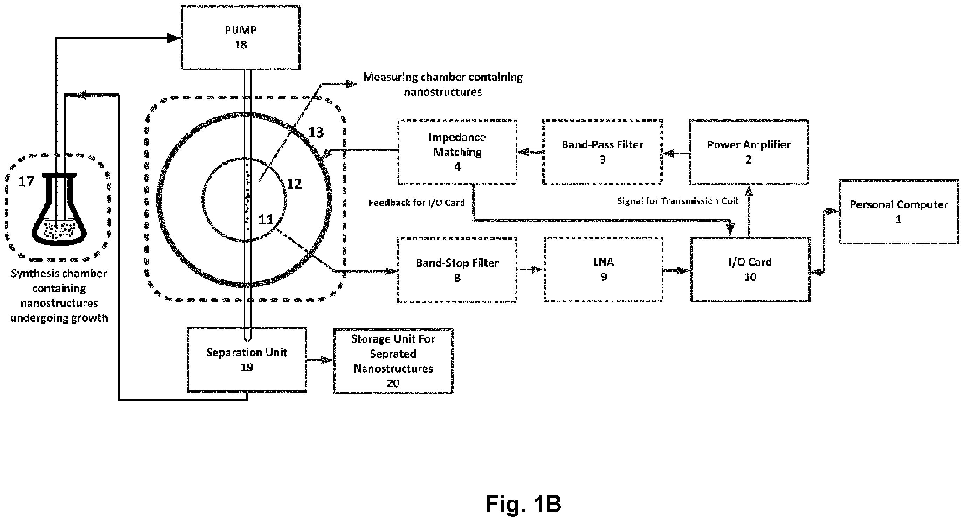

Fig. 1B shows a block diagram of another variant of the sensing device according to Fig. 1A. Instead of synthesizing the nanostructures inside the sensing apparatus, the nanostructures are pumped into the measurement chamber by a pump 18. The sensing device also comprises a separation unit 19 and a storage unit 20. The details of the separation unit 19 will be explained in details further below.

The difference between the devices according to Figs. 1A and 1B is that instead of having the synthesis process inside the sensing apparatus, a pump 18 is used to channel a small quantity of the nanostructures undergoing synthesis into the sensing device for measurement and then return it to the synthesis chamber 17 for further growth.

The amplitude and phase spectra of the received voltage signal are displayed for evaluating the nanostructures on a screen of the personal computer 1. Furthermore, the hysteresis curve is visualized on the screen of the personal computer 1. The amplitude spectrum contains both the even and odd harmonics. The odd harmonics are attributed to the signals from the nanostructures under the synthesis process. A static magnetic offset field such as the earth magnetic field induces the even harmonics in the signal. The amplitude spectrum or the time signal is the characteristic fingerprint of the nanostructure and is dynamic in the synthesis process. The complete nucleation and growth curve acquired during one synthesis of the nanostructures is shown further below for illustration, as well as a measurement protocol and a brief introduction to the synthesis process with some of the measurements obtained at different time points.

### Techniques for steering the magnetic properties of nanostructures

There are at least two implementations for controlling the nucleation and growth of the nanostructures. One of the methods is to steer the growth of the nanostructures with the help of external magnetic fields. The external magnetic fields could be a) spatially varying, static fields, and b) homogenous, dynamic (time-varying) fields, and c) spatially varying, dynamic (time-varying) fields. All these fields can further be defined for the x, y and z directions. Another method is chemical steering, in which growth and nucleation are manipulated by adding different reagents with adapted flow rates at different time steps. Both methods are now described in more detail.

### Steering the growth with external magnetic fields

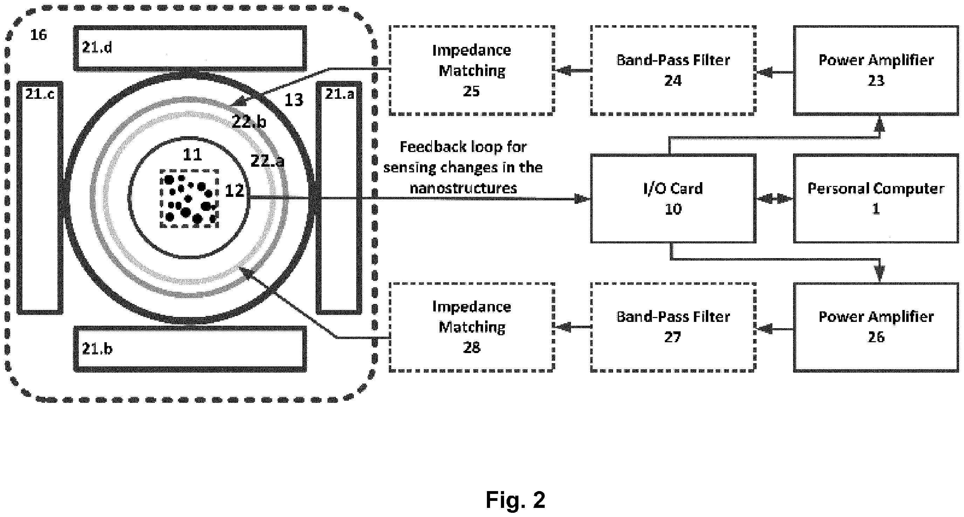

Fig. 2 shows an apparatus according to the invention for steering the growth of nanostructures with the help of external magnetic fields. This device is used in addition to the sensing apparatus described above. The device comprises different coil pairs 21a-d, 22a-b for generating the required magnetic field, followed by an impedance matching unit 25, 28 for transmission of maximum power from the power amplifier, a band-pass filter for attenuating the distortions produced by the power amplifier, and a power amplifier 23, 26 for generating the required field. The I/O card 10 generates the excitation signal supplied to the coils 12, 13 as well for acquiring the measurements from the nanostructures under synthesis. A personal computer 1 is used for displaying the data.

In more detail, the hardware of the device according to Fig. 2 generates static and dynamic fields for different directions. By the information provided by the sensing apparatus, the growth and nucleation of the nanostructures are steered by different external fields simultaneously or in stand-alone configuration with different coil topologies.

The device according to Fig. 2 works in combination with the sensing apparatus described above to provide an appropriate feedback loop. External fields are used for manipulating and steering the nucleation and growth of the nanostructures in a desired direction. Due to the presence of these external fields both the physical and magnetic properties can be manipulated. Here the physical properties include the shape, size, and size distribution, and the magnetic properties include the magnetic moment.

The hardware can be located inside the sensing apparatus (as shown in Fig. 2) or can be located at a distance as shown with the help of the separation unit 19 in Fig. 1B. The added advantage of having the external magnetic fields at a distant location is that these external fields can also be used for separating nanostructures according to their desired magnetic and physical properties. One of the principal applications is to separate the nanostructures which have attained the desired properties from the synthesis chamber 11 and store them in a storage unit 20. The rest of the particles is returned to the synthesis chamber 11 for further growth.

The various magnetic fields that can used are:
- Magnetic fields in the form of uniform homogenous static fields, generated in such a way that they act on the nanostructures in a synthesis process. Such an effect can be achieved with permanent magnets 21a, 21c in x direction, 21b, 21d in y direction and 22a in z direction).
- Magnetic field in the form of spatially varying static fields, generated in such a way that they act on the nanostructures in a synthesis process.
- Magnetic fields in the form of homogeneous, dynamic (time-varying) fields, generated in such a way that they act on the nanostructures in a synthesis process.
- Magnetic fields in the form of spatially varying, dynamic (time-varying) fields, generated in such a way that they act on the nanostructures in a synthesis process. This is performed for all directions with the help of a pair of orthogonal coils 21a, 21c in x direction and 21b, 21d in y direction and a solenoid 22a, 22b in z direction.

For producing the dynamic fields (homogenous/spatially varying) in x, y and z direction, there is a need to have additional power amplifiers 23, 26, band-pass filters 24, 27 and the required impedance matching 25, 28 for reducing the power losses between the coils and the amplifier. For illustration purpose only the components for x and y direction are shown, but the z direction also requires the extra components in the form of power amplifiers, band-stop filters and the required impedance matching. The function of the I/O card 10 is to provide the excitation signal to all the electromagnetic coils mentioned in the previous paragraph and to acquire the signals obtained through the receiving coil 12. The personal computer 1 is used for displaying the data obtained from such a synthesis process. The topologies of the electromagnetic coils can comprise saddle coils, solenoids, surface coils, etc.

The details for such a device as well as the initial results obtained can be seen in DE 10 2017 108 737 A1.

### Steering the growth with chemical and/or biological reagents

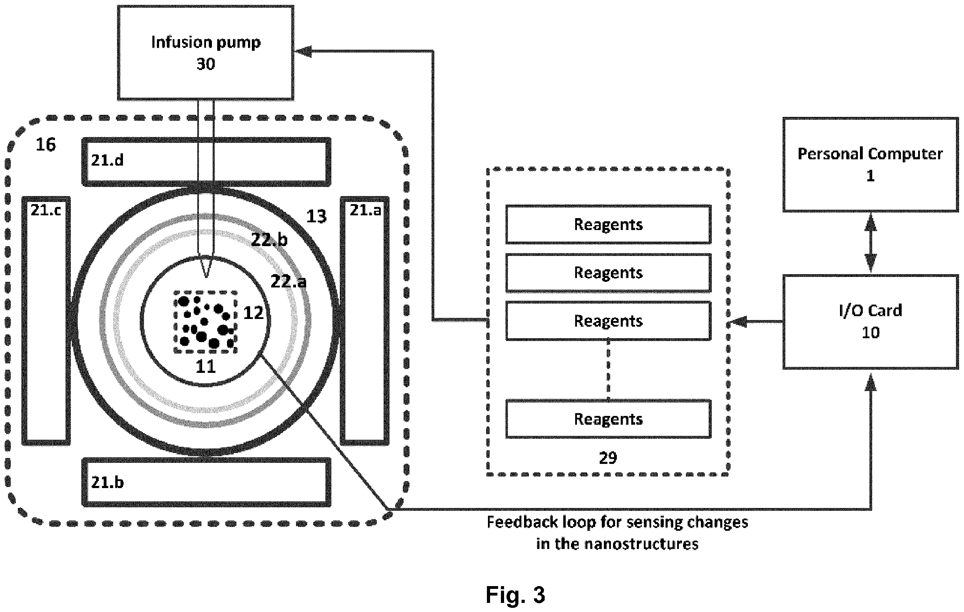

Fig. 3 shows a device according to the invention for steering the growth of nanostructures with chemical/biological reagents. The device comprises different coil pairs 21, 22 for generating the required magnetic field similarly to Fig. 2. Chemical and/or biological reagent infusion pumps 29, 30 controlled with the help of an I/O card 10 are used. The I/O card 10 senses the changes in the nucleation and growth pattern in the form of a feedback loop, administers the required quantity of the reagents, and monitors the growth of the nanostructures.

The nucleation and growth process can be adapted by the flow rate of the chemicals and reagents. The method applied for this purpose depends on the time constants of the sensing apparatus, as the chemical and/or biological reagents react fast. This steering method can be used in addition to the method using external magnetic fields described above.

Fig. 3 shows a hardware realization for such a device comprising an infusion pump 30 and a delivery mechanism 29. The I/O card 10 is used for controlling the infusion pump 30. The I/O card 10 senses the change in the magnetic properties of the nanostructures under synthesis and administers the required reagents in the required quantity to steer the nucleation and growth process. For different synthesis processes, the time points for the administration is an important steering parameter.

### Technical specification of the device

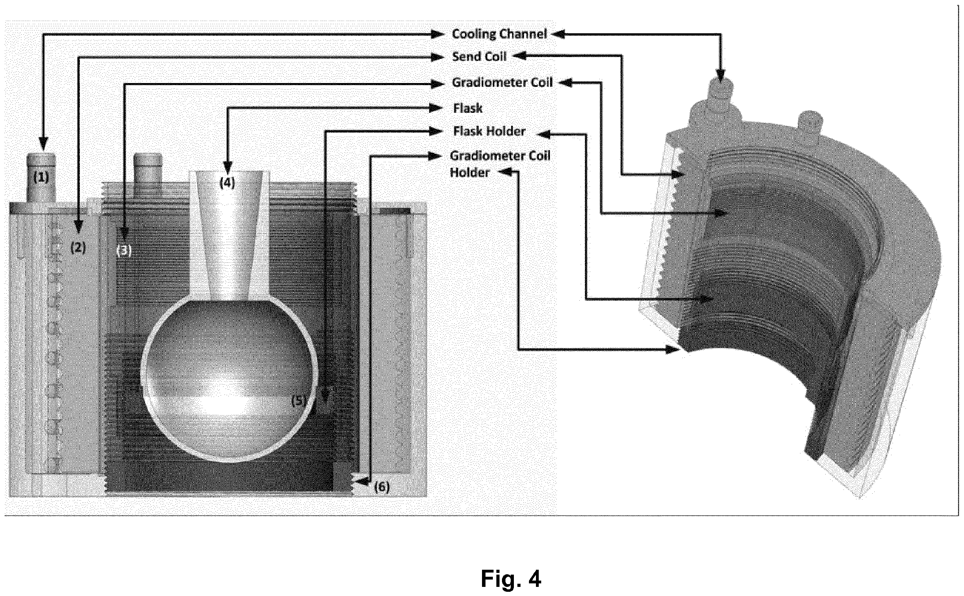

Fig. 4 shows a cross-section through the housing of a sensing device, comprising a cooling channel 1 to circulate air in and around the transmitting coil and the receiving coil, a transmitting coil 2, a receiving coil/gradiometer coil 3, a flask for the nanostructures 4, a flask holder 5, and a holder 6 for the receiving coil 3, providing the flexibility to move the gradiometer coil 3 for adapting for different synthesis processes.

Housing: The housing has four basic functions in the device and is an integral part of the device. The functions are as follows:
(A) for holding the transmitting coil 2 and the receiving coil 3;
(B) for accommodating a holder 5 designed for the measurement flask for the nanostructures being synthesized;
(C) for accommodating an air conduction system around the transmitting coil 2 and the receiving coil 3 for cooling the required coils. In the depicted embodiment, cooling is done with the help of pressurized air, but water or a coolant can also be used.
(D) for tuning the system, as there is a possibility to move the receiving coil 3 and the flask holder 5 to get a maximum signal from the receiving coil 3, therefore being adaptable for different reactions.

Power Amplifier: Any power amplifier which can produce the required field for exciting the nanostructures can be used. For the apparatus depicted in Fig. 4 an excitation field of 10 mT at a frequency of 23 kHz is used. One essential parameter for selecting the power amplifier is the total harmonic distortion (THD). Higher THD values mean that the excitation signal will contain other harmonics and add noise to the whole measurement process. This may lead to an addition of the band-pass filters in the transmitting chain. For the apparatus depicted in Fig. 4, a power amplifier from AE Techron 2015 is used.

impedance matching: Impedance matching consists of two capacitors Cₛ (in series) and Cₚ (in parallel). The function of the impedance unit is to transmit maximum power from the power amplifier to the transmitting coil.

Transmitting coil: In the apparatus depicted in Fig. 4 the transmitting coil 2 is responsible for producing the required field to excite the magnetic nanostructures. The topology of the transmitting coil 2 is a solenoid (other topologies can also be used such as saddle coils, flat circular coils etc., as shown in Fig. 1A) and comprises 40×4 windings realized with 2000×50 µm Litz wire. The coil is air cooled in the housing with pressurized air and is able to produce fields of around 10 mT.

Receiving coil: In the apparatus depicted in Fig. 4, a one-dimensional gradiometer coil is used to accommodate a receiving coil 3. Gradiometer coils have been used in number of fields such as for constructing magnetometers. The gradiometer coil comprises a receiving coil 3 and a cancellation coil (with reverse winding). The function of the cancellation coil is to attenuate the excitation signal which is coupled to the receiving coil 3. The gradiometer coil comprises pure copper wire with a diameter of 0.50 mm and has 30 turns in the receiving coil 3 and approx. 28 windings in the cancellation coil. The diameter of the gradiometer coil is 74 mm. Other configurations are also possible such as just using a receiving coil 3 followed by a band-stop filter to dampen the transmitting frequency in the transmitting coil 2 or a dedicated cancellation unit.

Signal generation and acquisition: For generation of the transmitting signal (Tₓ) and for acquiring the received signal (Rₓ), an I/O card featuring two 16-bit ADC (Analog to Digital Convertor) channels and two 16-bit DAC (Digital to Analog Convertor) channels is used. The sampling frequency for the Tₓ and Rₓ is 5.06 MHz. A PC is used with the I/O card as an interface to the device and for visualizing the measurements obtained from the nanostructures under observation.

### Experimental results from sensing apparatus

Synthesis process: There are number of synthesis processes for manufacturing nanostructures. These are usually divided into three major branches: alkaline precipitation in water, water-in-oil micro-emulsions, and thermal decomposition of organometallic iron in organic solvents. For validation of the sensing apparatus, alkaline precipitation in water is used. The corresponding details and the chemicals used for such a synthesis process is explained in Lüdtke-Buzug, K. (2012), 'Magnetische Nanopartikel', Chemie in unserer Zeit 46 (1), pp. 32-39.

Measurement protocol: Before initiating the synthesis process for nanostructures, an empty measurement is acquired, which is subtracted from the subsequent measurements to correct the background noise. Measurements are taken every 5 min over the entire duration of the synthesis process, but can be adapted according to the synthesis process. The total measurement time for one measurement is about 1 s, acquiring 10 periods with 2300 averages.

Measurements: The voltage signal induced in the receiving coil 3 can be divided into both the amplitude and the phase spectra, and in-turn hysteresis curves can be calculated. The amplitude spectra contain both the even and the odd harmonics. The odd harmonics are attributed to the signals from the nanostructures under the synthesis process. The even harmonics are caused by the Earth's magnetic field. The amplitude spectrum is the characteristic fingerprint of the nanostructure and is dynamic in the synthesis process.

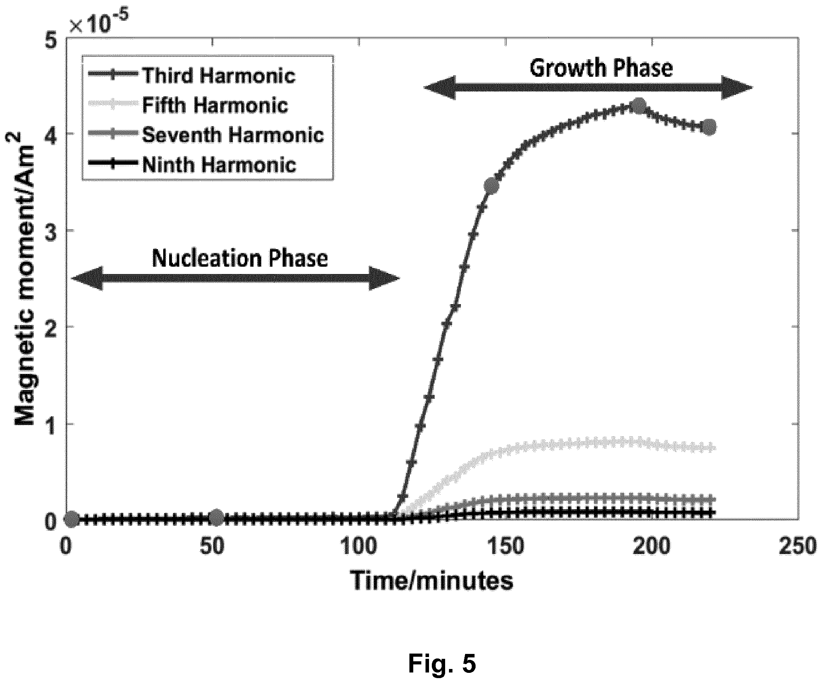

The complete nucleation and growth curve acquired during the synthesis of nanostructures is shown in Fig. 5 for illustration. The parameters and the synthesis process are explained above. The measurement consists of the change in the magnetic moment of the nanostructures versus time starting from third odd harmonic to ninth harmonic.

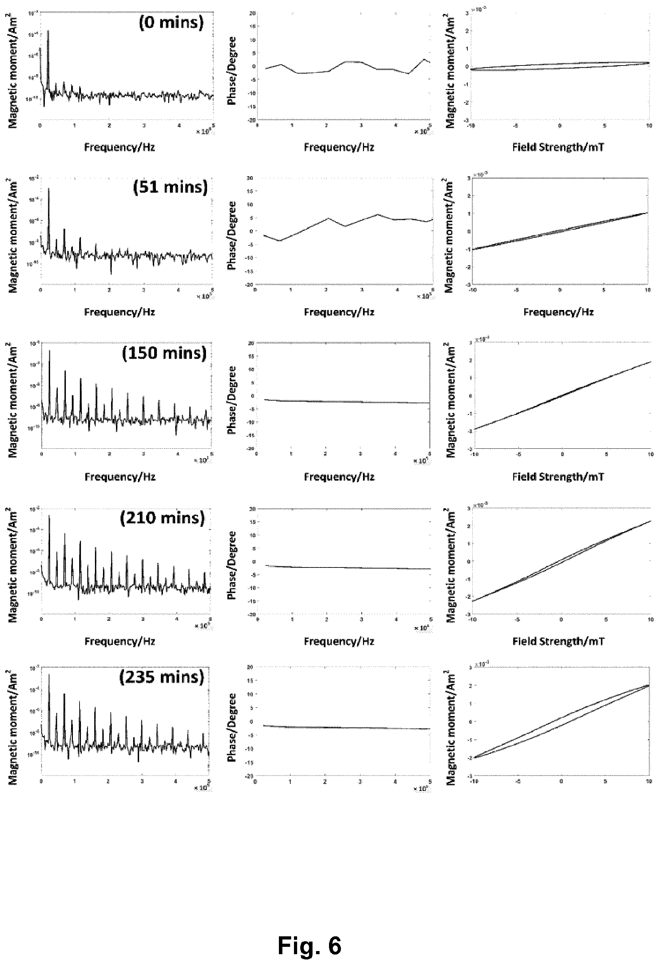

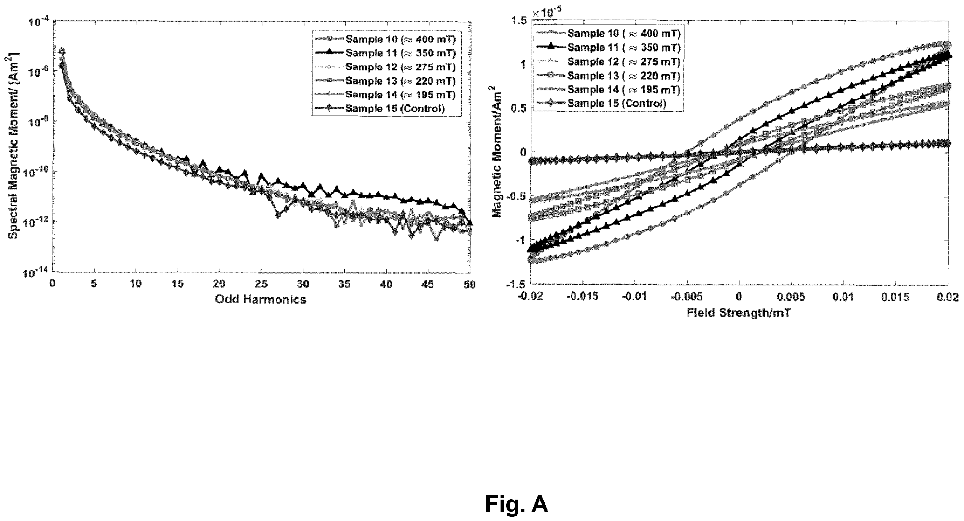

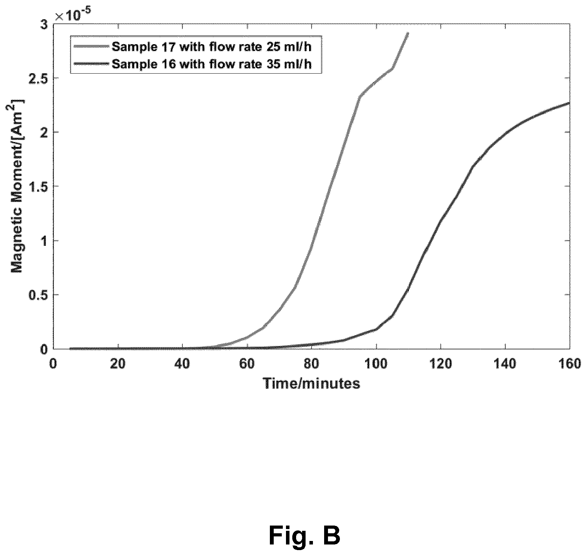

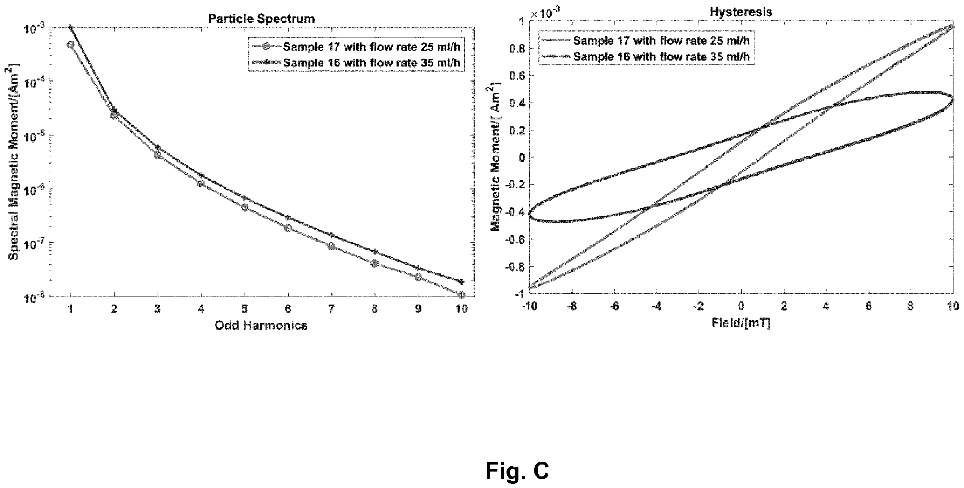

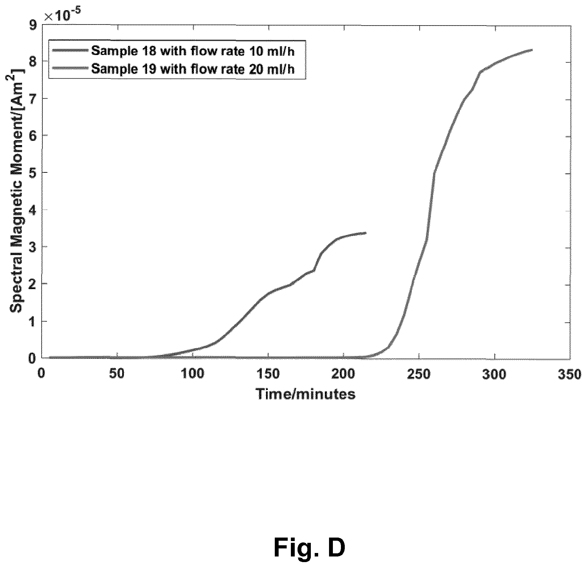

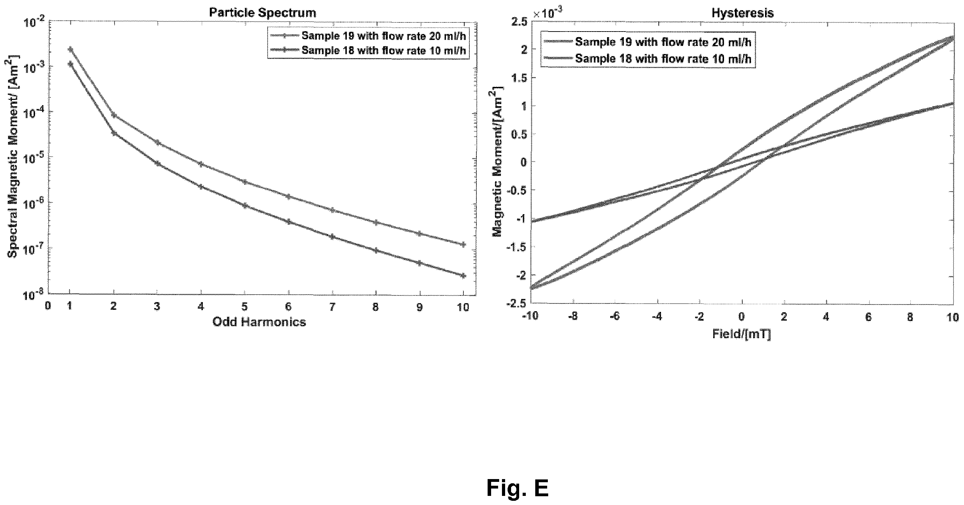

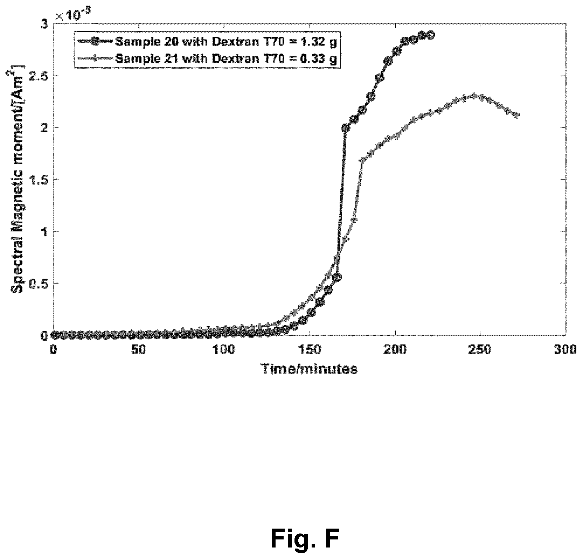

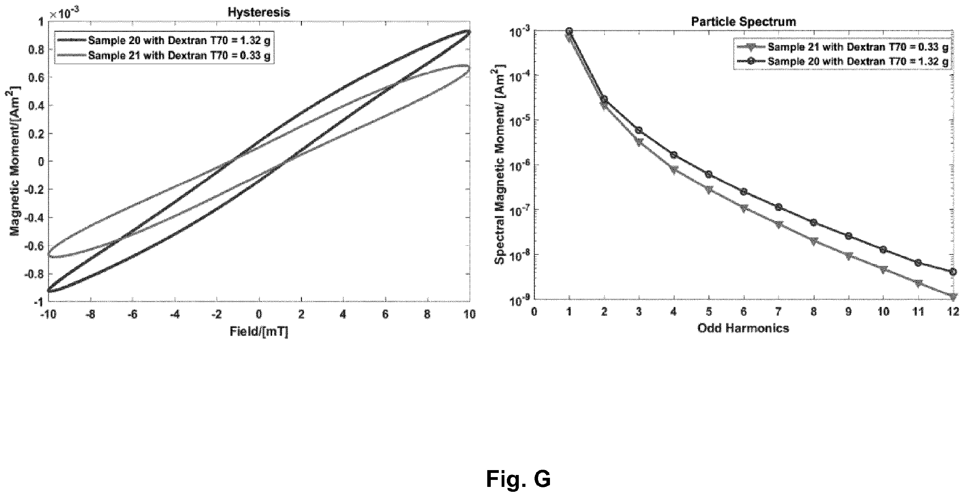

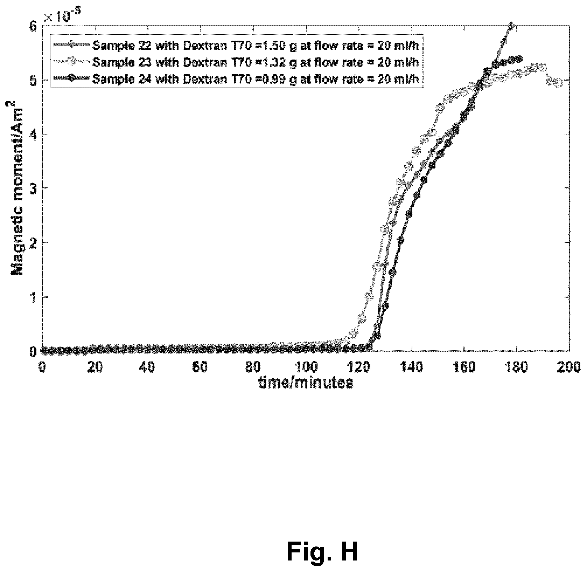

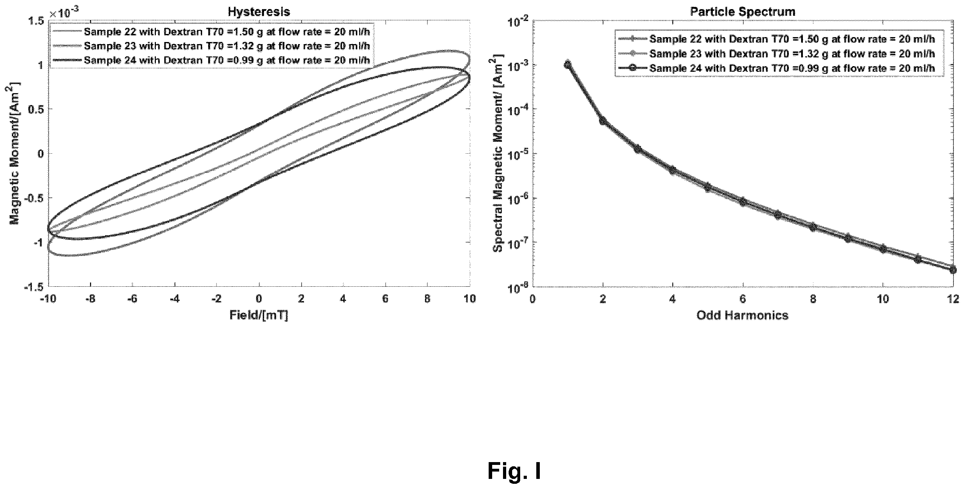

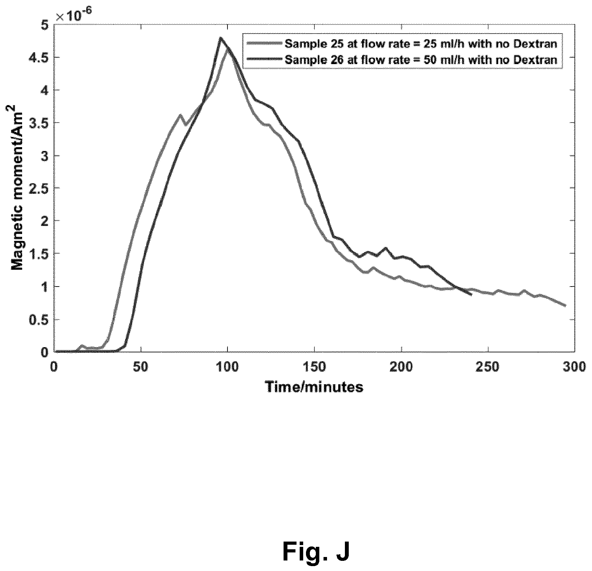

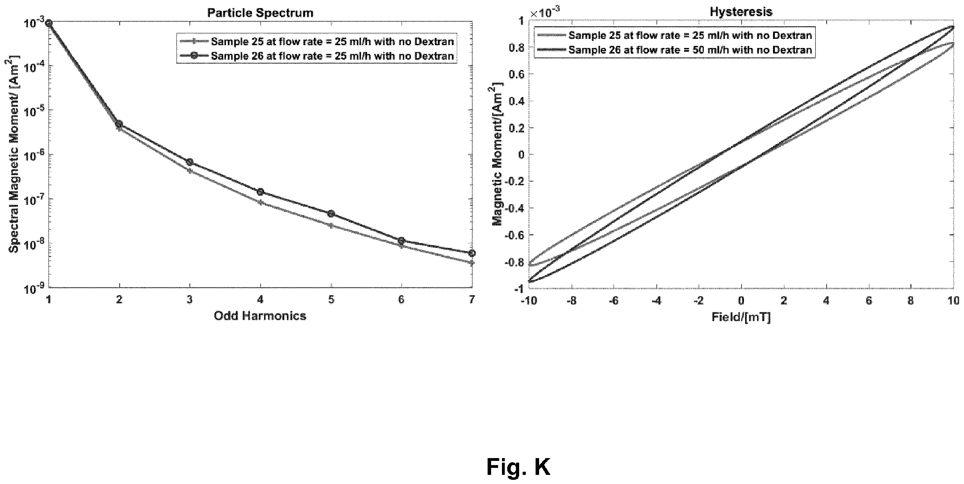

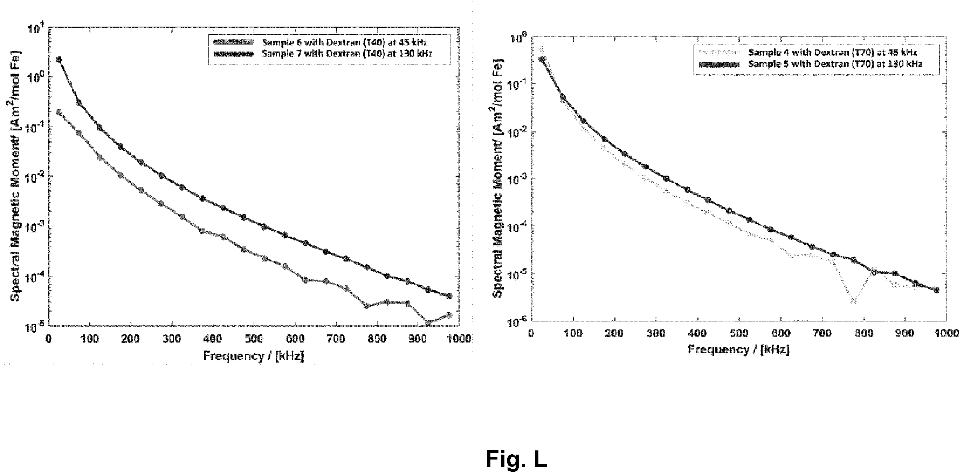

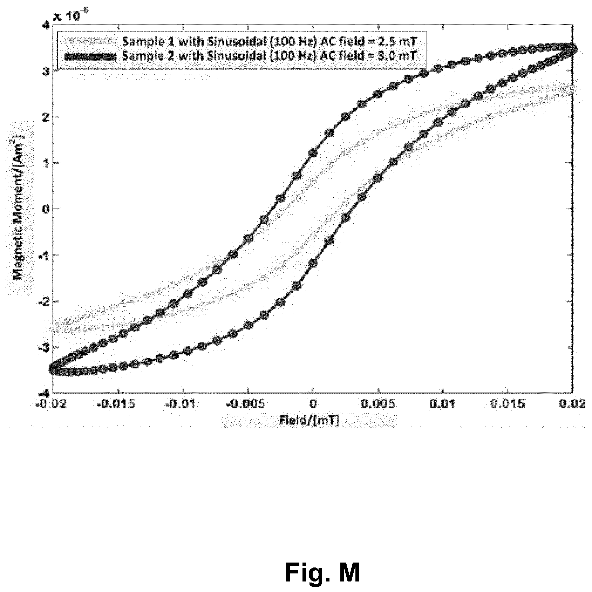

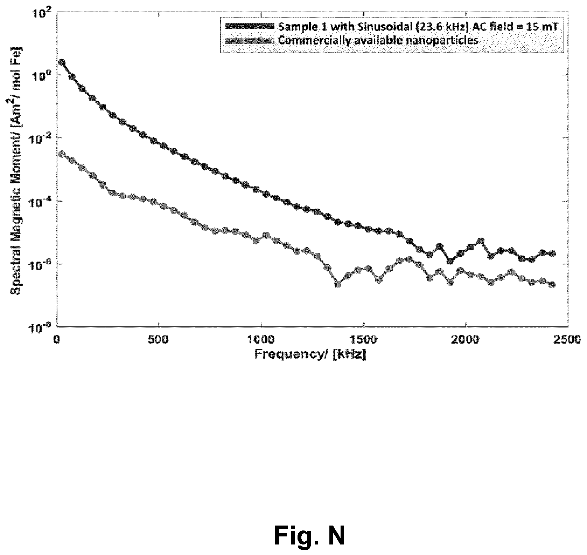

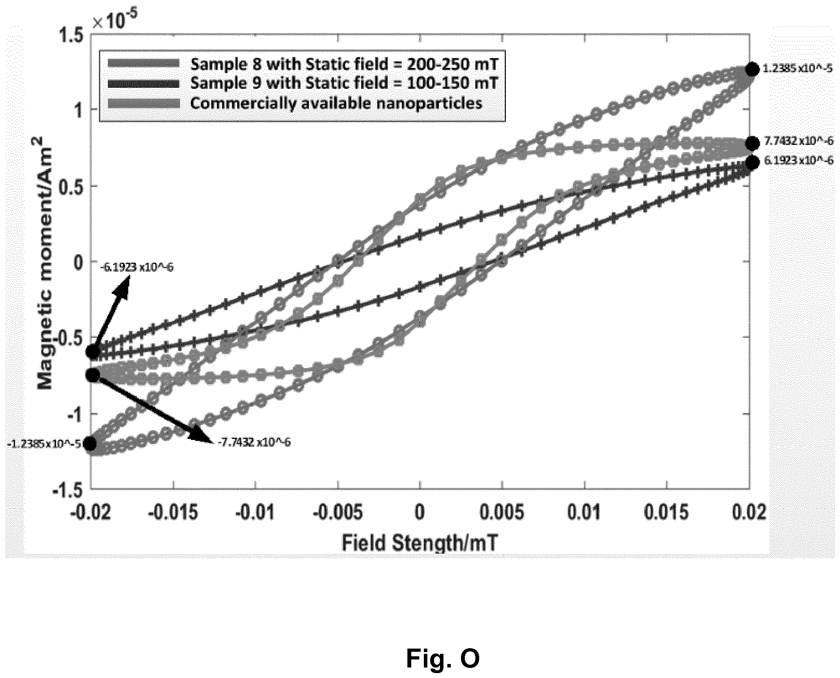

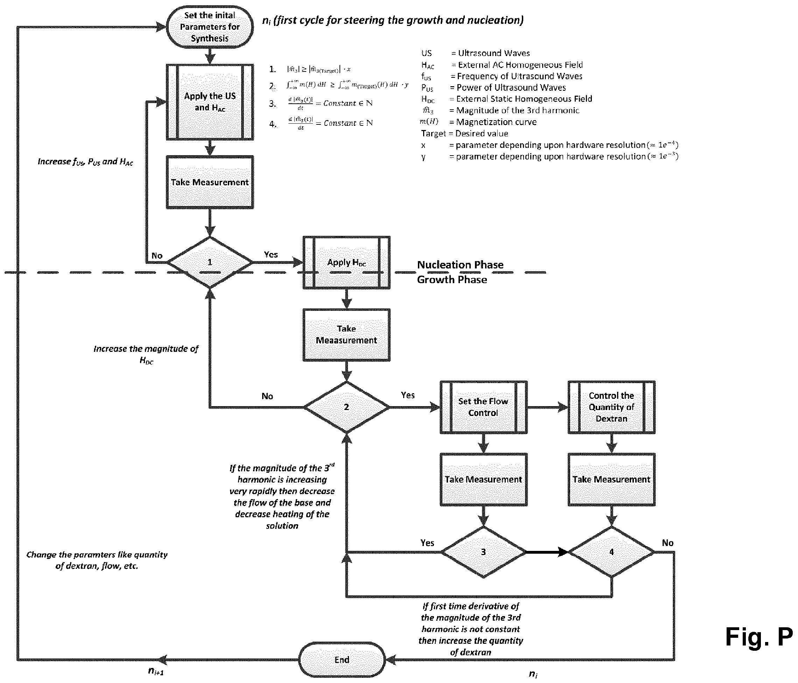

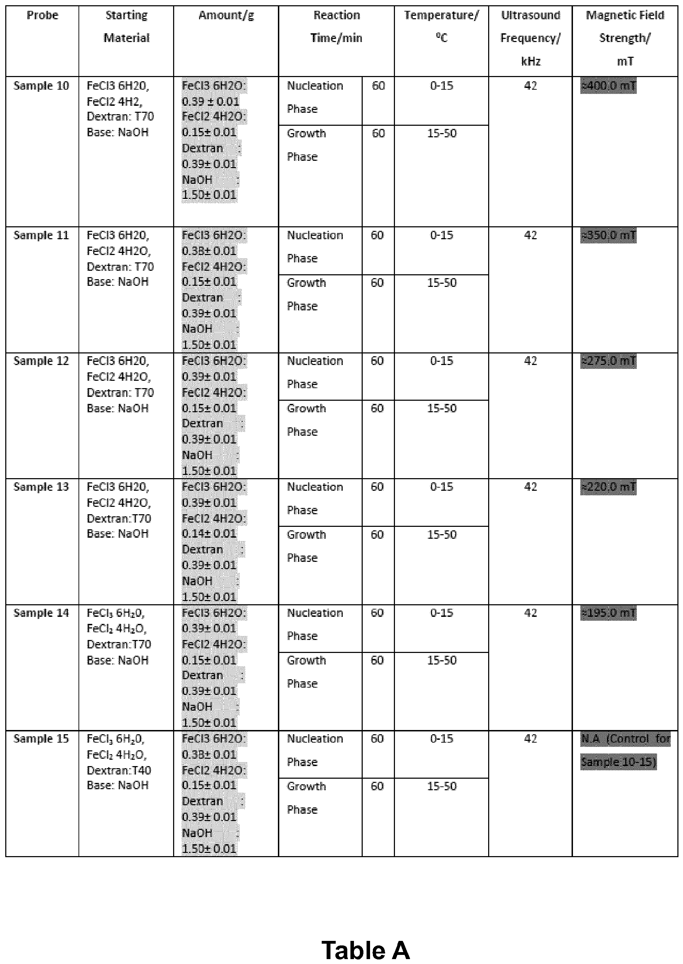

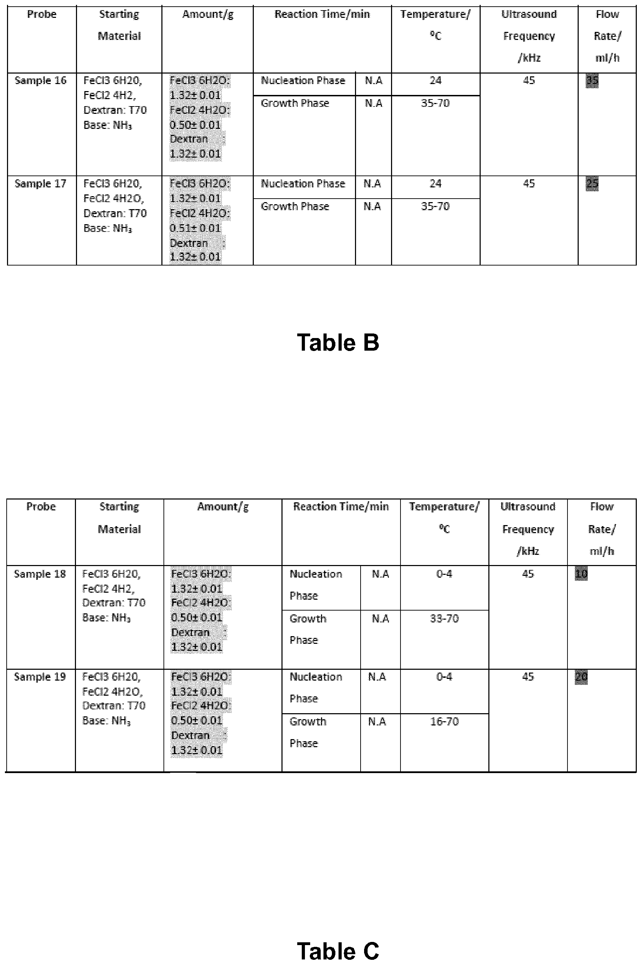

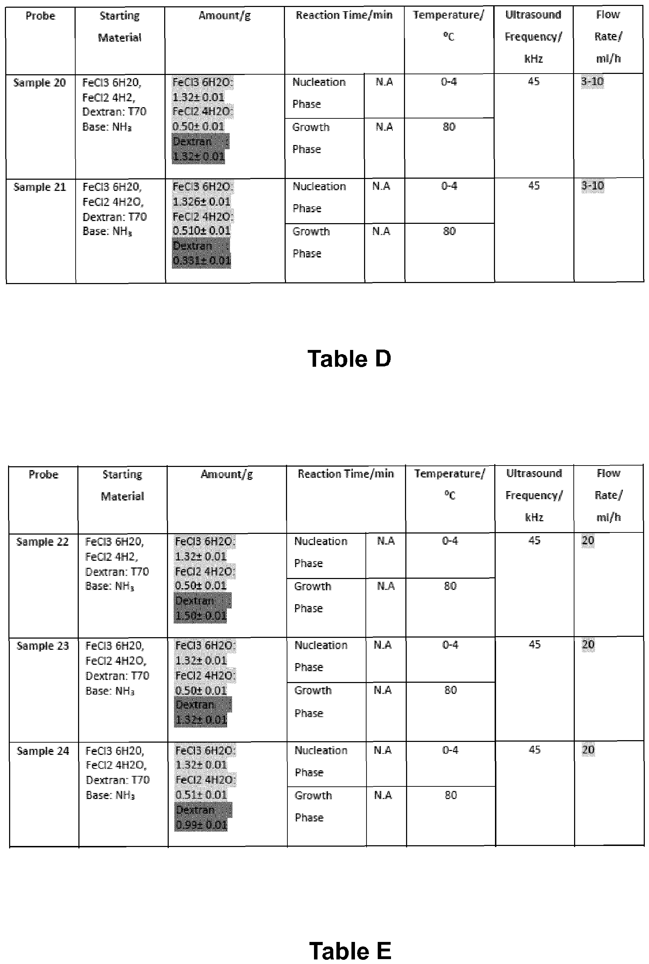

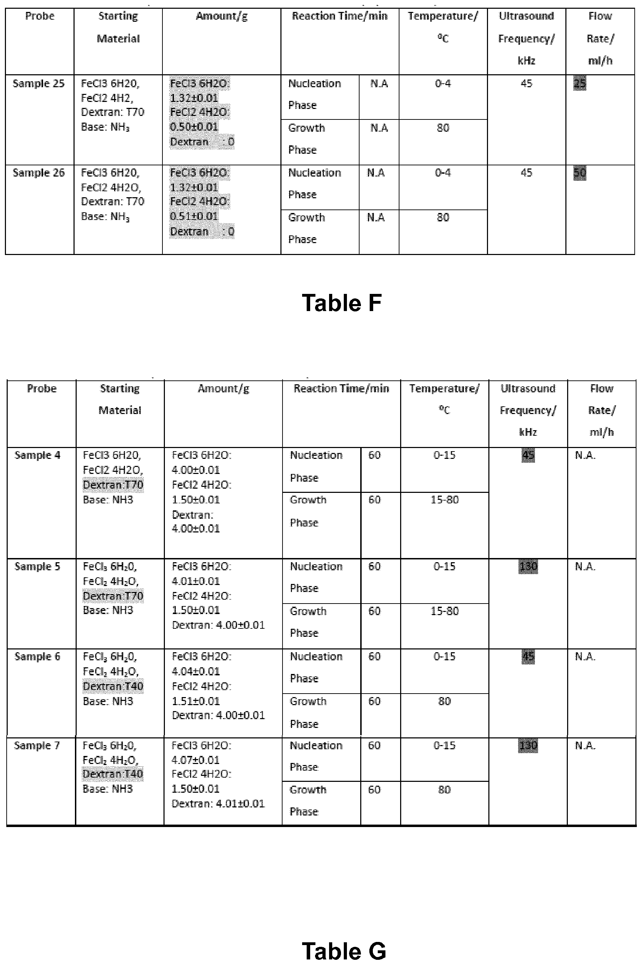

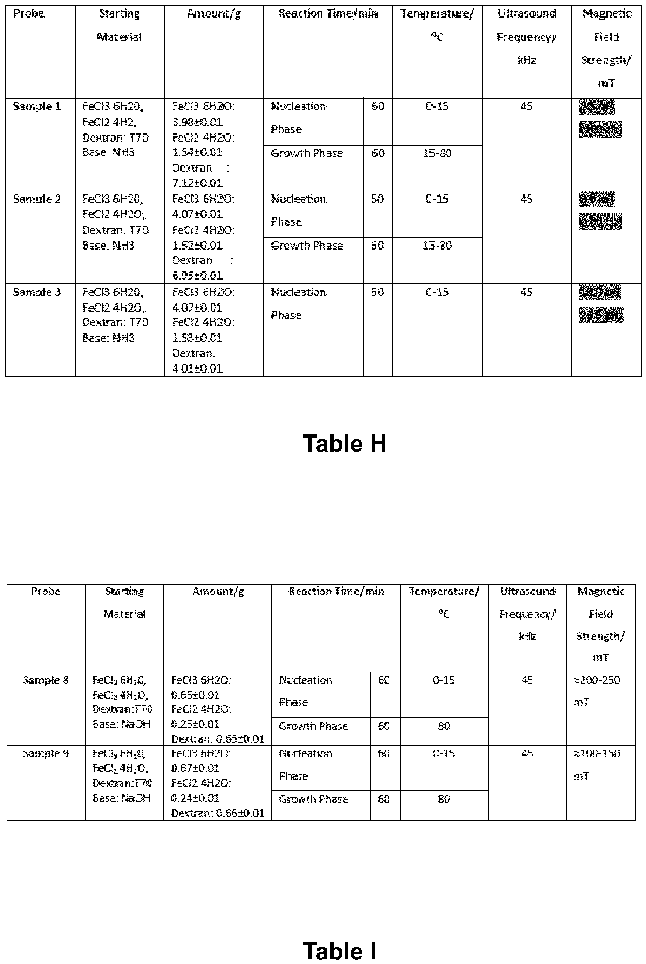

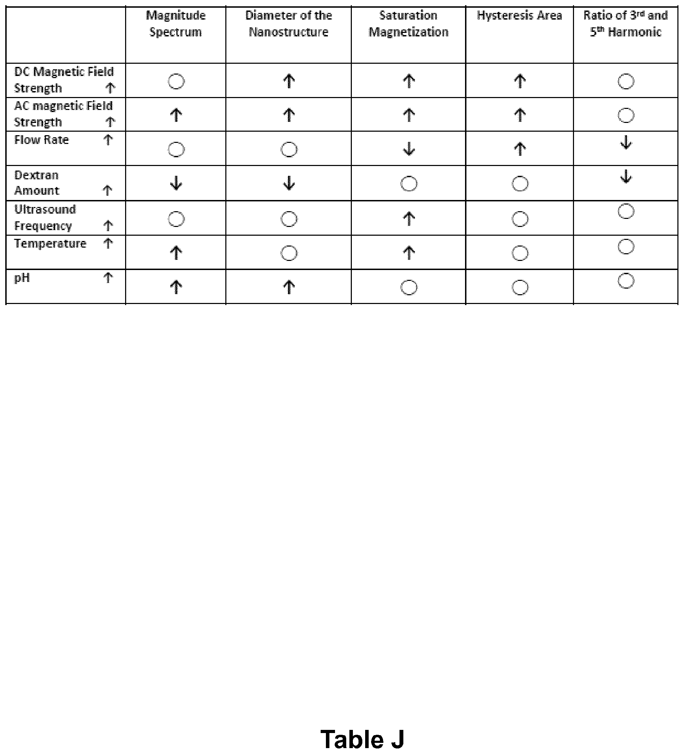

The points marked by large dots in Fig. 5 are further shown in Fig. 6 with their respective amplitudes and phase spectra and the calculated hysteresis curves obtained from respective measurements. The measurements and the curves can vary depending upon the parameters in the process of fabrication of the nanostructures. The duration of the illustrated measurement is approx. 220 min, and the data is acquired every three minutes and comprises around 74 measurements.

In more detail, Fig. 5 shows the magnetic moments of the third, fifth, seventh, and ninth harmonics versus the time of the entire synthesis process. The synthesis process can be subdivided into two different intervals. The first interval (from 0 to approx. 110 min) is the nucleation phase, where only a slight change in the magnetic properties can be seen over time. The second interval (from approx. 110 to approx. 235 min) is the growth phase, where a slow linear particle growth takes place which converges into a static behavior. The synthesis process is stopped in this phase.

In more detail, Fig. 6 shows the measurements of the amplitude and phase spectra for the points marked by large dots in Fig. 5. The first measurement row comprises the measurements taken at the starting of the synthesis process (0 min), followed by measurements taken at 51 min, 150 min, 210 min and the final measurement (at 235 min). The first two columns comprise the change in the amplitude and phase spectra in an ongoing synthesis process. In the third column, the calculated hysteresis curve is shown for the various time points.

### Experimentation

To evaluate the possibility of steering the properties of the magnetic nanostructures, experiments were performed to analyze the effect of various parameters (such as the presence of the external magnetic field, the change in the flow rate of chemicals and reagents as well as different quantities of coating materials) mentioned above. The details of the experiments and the parameters used are described below.

Synthesis of all the nanostructures was realized by an alkaline co-precipitation process. The co-precipitation technique is a straightforward process to obtain a substantial yield of magnetic nanostructures. Through this process, iron-oxides (Fe₃O₄) nanoparticles could be easily prepared by including a mixture of ferric and ferrous salts in an aqueous solution and precipitating with a base (NH₃, CH₃NH₂, NaOH etc.). The formation of nanostructures happens when the supersaturation is reached. A supersaturated solution contains a higher amount of the solute than the amount of solvents that it can dissolve. Moreover, other kinds of iron salts, bases and coating materials can be used for the synthesis of nanostructures.

The co-precipitation process comprises two stages: a short burst of nucleation, once the concentration of the species reaches critical supersaturation.

2*Fe*³⁺ + *Fe*²⁺ + 8 *OH*⁻ → (*OH*)₂ + 2*Fe*(*OH*)₃,

followed by a steady growth of the nuclei through the diffusion of the solutes to the surface of the nanostructures,

*Fe*(*OH*)₂ + 2*Fe*(*OH*)₃ → *Fe*₃*O*₄ + 4 *H₂O .*

Basically, for all the experiments the iron salts iron (III) and iron (II), dextran and demineralized water were placed in a flask in an ice bath, followed with the addition of a base at a constant rate under ultrasonic control. Iron (III) (FeCl3.6H2O, ≥ 99% obtained, from Carl Roth GmbH Karlsruhe, Germany) and Iron (II) (FeCl2.4H2O, ≥ 99% obtained, from Merck kGaA, Darmstadt, Germany) were used. Dextran T70 used for the experimentation was obtained from AppliChem GmbH, Darmstadt, Germany.

The flow rate of the base was controlled with the help of an infusion pump (PERFUSOR secura FT, B. Brown). The temperature of the reaction mixture was measured continuously with a fiber optic thermometer (FOTEMP 4, OPTOCON AG). This comprised the nucleation phase followed by the slow growth. In the growth phase, the mixture was heated to a definite temperature. For different experiments, these varied parameters are shown in the corresponding tables. For some of the experiments the sensing apparatus mentioned above was used for characterization (these measurements always have a change in magnetization versus time). For other measurements, a magnetic particle spectrometer (1D MPS) operating at 25 kHz with a magnetic field strength of 20 mT was used.

### Experiment I (Synthesis of super-paramagnetic iron oxides nanostructures subjected to static magnetic fields)

For synthesis, the iron salts and the dextran were dissolved in demineralized water at room temperature inside a flask. Then the flask was placed in an ultrasound bath with a frequency of 42 kHz. A pair of permanent magnets was used to generate the required magnetic field. The distance between the permanent magnets could be appropriately adjusted to attain the required magnetic field strength. For all the experiments a constant flow rate of 15 ml/h was used. The quantity of the different chemical reagents used as well as the external magnetic field strength in which the synthesis was performed are shown in Table A. In Sample 10-14 the external magnetic field strength was varied from approx. 400 mT to 195 mT, and Sample 15 plays the role of the control experiment, i. e. without any external magnetic field (B = 0 mT). For characterization, 1D MPS was used. Some initial experimentation was conducted to check the influence of the static magnetic field, and the measurements (Sample 8 and Sample 9) obtained are shown in Table I.

Fig. A shows the spectral magnetic moments and the hysteresis curves of all the synthesized samples at different field strengths measured with 1D MPS.

Conclusion: The amplitude spectrum of the different nanostructures does not change significantly with the change in the field strength. However, there is a significant impact on the hysteresis curves obtained. It is conjectured that the change in the hysteresis curve is due to an increase in the core size of the nanostructures in comparison to the control measurement. With the exception of the synthesis at 275 mT, the saturation magnetization increases linearly with an increasing magnetic field strength. Therefore, when the synthesis of nanostructures is performed in the presence of an external magnetic field, the hysteresis loop consists of a larger area. Moreover, magnetization saturation occurs at a higher level depending upon the field strength of the external magnetic field during synthesis.

### Experiment II (Synthesis of super-paramagnetic iron oxides nanostructures subjected to different flow rates of the base)

For synthesis, the iron salts and the dextran were dissolved in demineralized water at room temperature inside a flask and placed in an ultrasound bath with a frequency of 42 kHz. Moreover, the whole synthesis process was done in the sensing apparatus according to Fig. 1A. The measurements were acquired at different time steps. Every measurement took approx. 1 s. The field strength of the sensing apparatus was approx. 10 mT, which has a negligible effect on the growth and nucleation phase of the nanostructures, as only continuous application of high external magnetic fields has an impact on the nucleation and growth of the nanostructures. To sense the impact of the flow rate, this experiment was further divided into two experiments. The details of the experiments and the different parameters used are shown in Table B and Table C.

### Change in flow rate with no initial cooling

The addition of the base takes place in the presence of an ice bath to reduce the dynamics of the chemical reaction in the nucleation phase. For this particular experiment, no initial cooling was applied and only the flow rate was changed. The change in the flow rate is achieved with the help of an infusion pump (PERFUSOR secura FT, B. Brown). Sample 16 has a flow rate of 35 ml/h, and Sample 17 has a flow rate of 25 ml/h. Other parameters like the amount of iron salts and dextran were kept constant. Table B describes the different parameters in detail. For characterization, the change in the magnetic properties of the colloidal solution of the entire synthesis process is shown in Fig. B. Moreover, the amplitude and magnetization curve of the final measurement, are compared in Fig. C. All the measurements were acquired with the help of the sensing apparatus according to Fig. 1A.

Conclusion: It can be easily inferred from Figs. B and C that a higher flow rate leads to more delay in the initialization of the growth phase (as shown in Fig. B for Sample 16). But a smaller flow rate leads to a steeper increase in the magnetic moment (as shown in Fig. B for Sample 17 at a flow rate of 25 ml/h). Moreover, it leads to a higher magnetization saturation (as shown in the hysteresis curves of Sample 17 compared to Sample 16). These effects are more evident as there was no initial cooling.

### Change in flow rate with initial cooling

In this experiment, the flow parameter was again changed but with the initial cooling. So instead of starting the synthesis process at room temperature, the nucleation phase was started at a temperature ranging from 0 - 4 ⁰C. This initial cooling decreases the kinetic time constants of the reaction and may lead to a uniform formation of nuclei for growth. For this particular experiment flow rates of 10 ml/h (Sample 18) and 20 ml/h (Sample 19) were compared. All the other parameters were kept constant. The details of the parameters are stated in Table C. For characterization, the change in the magnetic properties of the colloidal solution of the entire synthesis process is shown in Fig. D. The amplitude spectra and magnetization curve of the final measurement are compared in Fig. E. All the measurements are acquired with the help of the sensing apparatus according to Fig. 1A.

Conclusion: It can be easily inferred from Figs. B and C that a higher flow rate leads to a delay in the initialization of the growth phase (as shown in Fig. D for Sample 19). These are consistent results compared with the previous experiment. But the steeper increase in the magnetization of Sample 20 is contradictory as compared to the previous experiment. This can be attributed to the initial temperature condition. Therefore, until and unless the chemical reaction reaches a specific temperature during the nucleation phase, there is no or insignificant growth happening. This provides the favorable conditions for the growth as seen in Sample 19 (in Figs. D and E). The nanostructures are able to grow significantly before the saturation kicks in.

The hysteresis curve, as well as the magnetic moment of Sample 18, is better for MPI/MFH than Sample 19, as shown in Fig. E. Hence, the role of the initial temperature in the nucleation phase cannot be neglected. Therefore, to obtain nanostructures which can be used for MPI or MFH, there is a need to maintain a balance between the flow rate and the temperature during the reaction.

### Experiment III (Effect of the quantity of Dextran on the nucleation and growth of the nanostructures)

This experiment was also divided into two subparts. In the first part, the amount of dextran was varied at lower flow rates of 3 - 10 ml/h, so the effect of the dextran could play a vital role. In the second part, the effect of the dextran was compared at higher flow rates of 20 ml/h.

### Change in amount of dextran at low flow rates (3 - 10 ml/h)

The amount of different chemical reagents used for the synthesis of nanostructures is shown in Table D. For this particular experiment two different quantities of dextran were used (Sample 20 contained 1.32 ± 0.01 g and Sample 21 contained 0.33 ± 0.01 g of dextran). All other parameters like the amount of iron salt, flow rate, etc. are kept constant. The results obtained through such an experiment are displayed in Figs. F and G. For this experiment the nucleation takes place at a lower temperature of 0 - 4 ⁰C.

Conclusion: From Fig. F it can be inferred that the amount of dextran plays a critical role in the growth phase of the nanostructures. The initialization of the growth corresponds to the amount of dextran in the chemical synthesis. At low flow rates of around 3 - 10 ml/h, it holds that the smaller the amount of dextran, the earlier the magnetic moment increases. This corresponds to the fact that the core of the nanostructures is growing faster. On the other hand, a higher amount of dextran leads to a more rapid and uniform growth and even causes a delay in the saturation effect. This can be clearly seen in Fig. F as Sample 20 with dextran T70 = 1.32 ± 0.01 g has a steeper slope than compared to Sample 21 with dextran T70 = 0.33 ± 0.01 g. Fig. G shows a comparison of amplitude spectra and hysteresis curves obtained from the final measurement.

### Change in amount of dextran at higher flow rates (20 ml/h)

In this setup of the experiment, the impact of the amount of dextran at a higher flow rate was investigated. Three different experiments were analyzed with different quantities of dextran. Sample 22 had approx. 1.5 ± 0.01 g of dextran, followed by Sample 23 and Sample 24 having approx. 1.3 ± 0.01 g and 0.99 ± 0.01 g of dextran, respectively. For these experiments, the flow rate was kept constant at 20 ml/h, and all other parameters like the quantity of iron salts were also kept constant. Detailed parameters are given in Table E below.

Fig. H shows the magnetic moment of the third harmonics versus time of the entire synthesis process for all three samples. Fig. I shows the amplitude spectra and the magnetization curve for all three measurements.

Conclusion: At higher flow rates the effect of the amount of dextran is not very evident, as the flow rate plays a much more essential role in achieving supersaturation for the chemical reaction. Hence, higher flow rates increase the possibility of the reactant to form magnetite. At higher flow rates the nucleation of the nanostructures is indecisive, the first nuclei formed do not follow the same pattern as described in the earlier experiment. This is shown in the zoomed image of the nucleation phase for all three samples.

Sample 22 and Sample 24 showed almost the same change in the magnetic movement (Fig. H), regardless of the quantity of dextran involved in the reaction. The saturation of the nanostructures occurred first in Sample 23 (Dextran = 1.32 ± 0.01 g) followed by Sample 24 (Dextran = 0.99 ± 0.01 g), and the nanostructures with the maximum quantity of dextran in Sample 22 (Dextran = 1.50 ± 0.01 g) never saturated. But if we compare a previous Sample 20 (Dextran = 1.32 ± 0.01 g and flow rate of 3 - 10 ml/h) with Sample 24 (Dextran = 1.32 ± 0.01 g and flow rate of 20 ml/h), the same inference can be drawn that the higher the flow rate, the later the nanostructures starts growing. In Sample 20 this effect can be seen at a time step of around 100 min. But at a higher flow rate (Sample 24), this occurs around the time step of 120 min. The amplitude spectra of all the samples are relatively equal in magnitudes. To evaluate this much further, there is a necessity to evaluate the effects of flow rate as well as the dextran separately. This is achieved through the comparison between Sample 20 and Sample 21, where the minimum flow rate of 3 - 10 ml/h is used. Furthermore, to confirm the effect of flow rate, Sample 25 and Sample 26 are synthesized without any dextran and will be shown in the next experiment.

### Experiment IV (Effect of flow rate on the nucleation and growth of the nanostructures without the presence of dextran)

For this particular experiment, the nanostructures were synthesized without the presence of dextran at different flow rates. The details of the parameters are shown in Table F. Sample 25 was synthesized with a flow rate of 25 ml/h, and Sample 26 was synthesized with a flow rate of 50 ml/h. All other parameters were kept constant. Fig. J shows the change in the magnetic moment versus time for both samples. Fig. K displays the magnetic spectra and hysteresis of the particles at the highest magnetic moment.

Before going into the comparison of the two samples, it is crucial to interpret the behavior of these curves. It can be seen from Fig. J that the nanostructures show an increase in magnetic moment till approx. 100 min in the synthesis chamber, and later on there is a decrease in the magnetic moment. This is caused by the absence of the coating material such as dextran. When the nanostructures achieve a critical size, the agglomeration between the nanostructures begins (as particle-particle interaction increases), and the field strength of the sensing apparatus is not high enough to influence the growth of these nanostructures. Hence, with the increase in agglomeration the size of the nanostructures increases and, with time, there is a constant decrease in the magnetic moment. Nevertheless, the same behavior is observed in the initial nucleation and growth of these nanostructures, i. e. the higher the flow rate, the later the growth begins. This can be easily inferred by comparing Sample 25 (flow rate of 25 ml/h), in which growth starts earlier in comparison to Sample 26 (flow rate of 50 ml/h). Fig. K shows the higher amplitude spectra for Sample 26 as compared to Sample 25, but for the hysteresis curve, there is a negligible difference.

### Experiment V (Effect of Ultrasound Frequency on the nucleation and growth of the nanostructures)

This experiment related to a comparison of ultrasound frequency with different types of dextran (T40 and T70) with two different frequencies (45 kHz and 130 kHz). As it could be easily inferred there is a difference in the amplitude of the signals produced in the magnetic particle spectrometer (MPS) for the measurement. The reason behind this is that ultrasound waves produce cavitation bubbles, and the size of these cavitation bubbles decreases with increasing frequency. When these cavitation bubbles burst, there is a release of energy leading to a mixing of reagents in the synthesis flask.

The effect of the ultrasound frequency is more differentiable with the usage of dextran (T40) than with dextran (T70). This difference could be due to the difference in the molecular mass of different dextran species. The details of the parameters are shown in Table G. Fig. L shows the amplitude spectrum of the above-mentioned synthesis. The magnetic moment of the nanostructures synthesized at a higher ultrasonic frequency is higher than compared to the synthesis which undergoes nucleation and growth at a lower ultrasonic frequency.

### Experiment VI (Effect of AC homogenous magnetic field strength on the nucleation and growth of the nanostructures)

For synthesis, the iron salts and the dextran were dissolved in demineralized water at room temperature. The flask was placed in an ultrasound bath with a frequency of 42 kHz. A homogenous sinusoidal magnetic field at 100 Hz was generated with the help of a solenoid around the synthesis flask. The magnetic field strength was varied with the help of a power amplifier.

For comparison purpose, in the first experiment a low field strength of 2.5 mT (Sample 1) and 3.0 mT (Sample 2) were compared. In the second experiment, Sample 3 (synthesized with a homogenous sinusoidal field at 23.6 kHz with a field strength of 15 mT) was compared with commercially available nanoparticles. The different parameters used for the synthesis are shown in Table H. Fig. M shows the comparison of the magnetization curve obtained through 1D MPS for Sample 1 and Sample 2. Fig. N shows a comparison between Sample 3 (field strength = 15 mT at 23.6 kHz) with commercially available nanoparticles.

Conclusion: Homogenous magnetic AC fields also have a prominent effect on the nucleation and growth of the nanostructures. This can be seen by comparing the magnetization curves between Sample 1 (sinusoidal 100 Hz at 2.5 mT) and Sample 2 (sinusoidal 100 Hz at 3.0 mT). Higher magnetic field strengths of the excitation signal lead to bigger core sizes of the nanostructures and hence, these particles are better suited for MFH. Sample 3 is synthesized not only at higher magnetic field strength (approx. 15 mT) but also with a higher excitation frequency signal (23.6 kHz). The amplitude spectrum obtained is higher in comparison to commercially available nanoparticles (Fig. N). Moreover, the number of visible harmonics goes beyond 1.5 MHz. This proves that the excitation frequency at the time of synthesis can influence the nucleation and growth of the nanostructures in a positive way. But to come to a conclusion, there is a need to do more experimentation with different field strengths at different frequencies and also different excitation signals like cosine, chirps, triangular, etc.

### Synthesis of super-paramagnetic iron oxides nanostructures subjected to static magnetic fields

Experiment I was previously conducted and the results obtained are completely consistent to Experiment I above. The parameters used are shown in Table I and the magnetization curves obtained are shown in Fig. O. Moreover, these nanostructures are also compared with the commercially available particles.

### Synthesizing nanostructures for future usage in medicine and biological science

There is no specific procedure to synthesize nanostructures and the parameters should be changed according to the application. All these parameters play a vital role in the genesis of the nanostructures with desired geometrical properties (size, shape and hydrodynamic diameter) and magnetic properties (amplitude spectrum and magnetization curves).

Some of the key parameters to consider in making ideal particles for MPI are stated below with their effects on the properties of nanostructures in Table J. In this table, the direction arrows mark an increase or decrease in the concerned properties. Circles mark no significant effect, or other parameters play a more vital role (specifically the flow rate).

That is, the properties which are marked with circles either have no effect on the final nucleation and growth of nanostructures, or it is difficult to differentiate the effects caused due to changing these parameters. For example, an increase in the DC magnetic field strength can lead to an increase in the diameter, the saturation magnetization and the area under the hysteresis, but has no significant impact on the magnitude spectrum or the ratio of third and fifth harmonics.

Flow Rate: This is one of the most significant parameters, as it controls the overall thermodynamics of the reaction. The ideal flow rate should be between 10 ml/h and 30 ml/h.

Quantity of Dextran: For synthesizing smaller nanostructures it is advisable to use large quantities of dextran, i. e. more than 1.32 ± 0.01 g, and then try to control other parameters through different means. For making bigger nanostructures the quantity of dextran should be reduced to less than 0.50 ± 0.01 g.

External Static Magnetic Field: Apply an external magnetic field to influence the diameter of the nanostructure core indicated by the enclosed area of the hysteresis curve.

External AC Magnetic Field: An external AC magnetic field affects both the amplitude spectrum and the enclosed area of the hysteresis curve. It should be applied for further controlling these parameters.

Ultrasonic Field: The ultrasound frequency also plays a vital part for the amplitude spectrum. A lower value of the ultrasound frequency (for example 45 kHz) gives a better magnetic amplitude response of nanostructures in MPS compared to a higher frequency (for example 130 kHz).

Nucleation and growth of nanostructures is a very complex process. The final characteristics of the nanostructures widely depend on a mixture of the above parameters. Hence, the best steering capabilities can be achieved when the synthesis process undergoes numerous cycles.

Fig. P shows a possible algorithm to control the nucleation and growth of the nanostructures in a continuous-flow synthesis process. For the initialization of synthesis, the parameters are chosen according to the chemical synthesis process, and then continuous measurements are performed to measure the physical properties (amplitude and phase spectrum and magnetization curves). Depending on these measured physical characteristics the parameters are adapted in the discretized control loop.

## Claims

1. Method for steering the growth of magnetic nanostructures,
wherein a method for sensing the growth of magnetic nanostructures is executed,
in which a method for sensing magnetic nanostructures with the steps of:
- providing magnetic nanostructures in a measurement chamber (11);
- exciting the magnetic nanostructures in the measurement chamber (11) by a magnetic field generated by a coil arrangement (12, 13);
- sensing the magnetic field in the measurement chamber (11) by the coil arrangement (12, 13);
- evaluating the magnetic nanostructures by using the sensed magnetic field;
is carried out at least at two different points of time at different stages during a growth process of the magnetic nanostructures,
and wherein additionally, depending on the evaluation of the magnetic nanostructures at a certain point of time, the growth of the magnetic nanostructures is influenced,
thus providing a feedback loop for steering the nucleation and growth process of the magnetic nanostructures,
**characterized in that**
the magnetic nanostructures reside in the measurement chamber (11) throughout the execution of the method for steering the growth of magnetic nanostructures
or
wherein at least a part of the magnetic nanostructures reside in the measurement chamber (11) during execution of the steps of the method for sensing the growth of magnetic nanostructures, and the growth of the magnetic nanostructures is influenced outside the measurement chamber (11).

2. Method according to claim 1, **characterized in that**, in the method for sensing magnetic nanostructures, the coil arrangement (12, 13) comprises a transmitting coil (13) and a receiving coil (12) which is different from the transmitting coil (13) or that the coil arrangement (12, 13) comprises a coil which acts both as a transmitting coil and as a receiving coil.

3. Method according to one of the preceding claims, **characterized in that**, in the method for sensing magnetic nanostructures, the excitation frequency of the magnetic field generated by the coil arrangement (12, 13) for exciting the magnetic nanostructures is attenuated, in particular by using a band stop filter (8) following the receiving coil, by using parallel sensing, by using a gradiometer coil as a receiving coil, or by a combination of these techniques.

4. Method according to one of the preceding claims, **characterized in that**, in the method for sensing magnetic nanostructures, the signal corresponding to the magnetic field sensed by the coil arrangement (12, 13) is amplified, in particular by a low noise amplifier (9).

5. Method according to one of the preceding claims, **characterized in that** the method for sensing the growth of magnetic nanostructures is carried out in real time, i. e. while the magnetic nanostructures are growing.

6. Method according to one of the preceding claims, wherein the growth of the magnetic nanostructures is influenced by applying an external field, in particular a magnetic, electrical, electro-magnetic, and/or ultrasonic field, to the magnetic nanostructures or by applying chemical and/or biological reagents to the magnetic nanostructures.

7. Method according to one of the preceding claims,
wherein at least a part of the magnetic nanostructures reside in the measurement chamber (11) during execution of the steps of the method for sensing the growth of magnetic nanostructures, and the growth of the magnetic nanostructures is influenced outside the measurement chamber (11) and
wherein at least a part of the magnetic nanostructures are pumped into or out of the measurement chamber (11) between the execution of the steps of the method for sensing the growth of magnetic nanostructures and the step of influencing the growth of the magnetic nanostructures.

8. Method for obtaining magnetic nanostructures, wherein the method for steering the growth of magnetic nanostructures according to one of the preceding claims is executed and wherein additionally at least a part of the magnetic nanostructures are separated from the remaining magnetic nanostructures on the basis of their physical and/or magnetic properties and are stored separately from the remaining magnetic nanostructures.

9. Device for steering the growth of magnetic nanostructures, adapted to execute a method for steering the growth of magnetic nanostructures according to one of claims 1 to 7, comprising
- a measurement chamber (11) adapted to accommodate magnetic nanostructures;
- a coil arrangement (12, 13) adapted to excite the magnetic nanostructures in the measurement chamber (11) by a magnetic field and to sense the magnetic field in the measurement chamber (11);
- an evaluation unit (1) adapted to evaluate the magnetic nanostructures by using the sensed magnetic field;
and an influencing unit (21, 22, 30) adapted to, depending on the evaluation of the magnetic nanostructures by the evaluation unit (1), influence the growth of the magnetic nanostructures.

10. Device for obtaining magnetic nanostructures, in particular adapted to execute a method for obtaining magnetic nanostructures according to claim 8, comprising a device for steering the growth of magnetic nanostructures according to claim 9, a separation unit (19) adapted to separate at least a part of the magnetic nanostructures from the remaining magnetic nanostructures on the basis of their physical and/or magnetic properties, and a storage unit (20) adapted to store these magnetic nanostructures separately from the remaining magnetic nanostructures.

## Patentansprüche

1. Verfahren zum Steuern des Wachstums von magnetischen Nanostrukturen,
wobei ein Verfahren zum Erfassen des Wachstums von magnetischen Nanostrukturen ausgeführt wird,
bei dem ein Verfahren zum Erfassen magnetischer Nanostrukturen mit den Schritten:
- Bereitstellen magnetischer Nanostrukturen in einer Messkammer (11);
- Anregen der magnetischen Nanostrukturen in der Messkammer (11) durch ein von einer Spulenanordnung (12, 13) erzeugtes Magnetfeld;
- Erfassen des Magnetfeldes in der Messkammer (11) durch die Spulenanordnung (12, 13);
- Auswerten der magnetischen Nanostrukturen anhand des erfassten Magnetfelds;
zu mindestens zwei verschiedenen Zeitpunkten in verschiedenen Stadien eines Wachstumsprozesses der magnetischen Nanostrukturen ausgeführt wird,
und wobei zusätzlich, abhängig von der Auswertung der magnetischen Nanostrukturen zu einem bestimmten Zeitpunkt, das Wachstum der magnetischen Nanostrukturen beeinflusst wird,
wodurch eine Rückkopplungsschleife zur Steuerung des Keimbildungs- und Wachstumsprozesses der magnetischen Nanostrukturen bereitgestellt wird,
**dadurch gekennzeichnet, dass**
die magnetischen Nanostrukturen sich während der gesamten Ausführung des Verfahrens zum Steuern des Wachstums magnetischer Nanostrukturen in der Messkammer (11) befinden
oder
wobei zumindest ein Teil der magnetischen Nanostrukturen sich während der Ausführung der Schritte des Verfahrens zum Erfassen des Wachstums magnetischer Nanostrukturen in der Messkammer (11) befindet und das Wachstum der magnetischen Nanostrukturen außerhalb der Messkammer (11) beeinflusst wird.

2. Verfahren nach Anspruch 1, **dadurch gekennzeichnet, dass** bei dem Verfahren zum Erfassen magnetischer Nanostrukturen die Spulenanordnung (12, 13) eine Sendespule (13) und eine von der Sendespule (13) verschiedene Empfangsspule (12) umfasst oder dass die Spulenanordnung (12, 13) eine Spule umfasst, die sowohl als Sendespule als auch als Empfangsspule wirkt.

3. Verfahren nach einem der vorhergehenden Ansprüche, **dadurch gekennzeichnet, dass** bei dem Verfahren zum Erfassen magnetischer Nanostrukturen die Anregungsfrequenz des von der Spulenanordnung (12, 13) zur Anregung der magnetischen Nanostrukturen erzeugten Magnetfeldes gedämpft wird, insbesondere durch Verwendung eines der Empfangsspule nachgeschalteten Bandstoppfilters (8), durch Verwendung einer Parallelerfassung, durch Verwendung einer Gradiometerspule als Empfangsspule oder durch eine Kombination dieser Techniken.

4. Verfahren nach einem der vorhergehenden Ansprüche, **dadurch gekennzeichnet, dass** bei dem Verfahren zum Erfassen magnetischer Nanostrukturen das dem von der Spulenanordnung (12, 13) erfassten Magnetfeld entsprechende Signal verstärkt wird, insbesondere durch einen rauscharmen Verstärker (9).

5. Verfahren nach einem der vorhergehenden Ansprüche, **dadurch gekennzeichnet, dass** das Verfahren zum Erfassen des Wachstums von magnetischen Nanostrukturen in Echtzeit, d. h. während des Wachstums der magnetischen Nanostrukturen, ausgeführt wird.

6. Verfahren nach einem der vorhergehenden Ansprüche, wobei das Wachstum der magnetischen Nanostrukturen durch Anlegen eines äußeren Feldes, insbesondere eines magnetischen, elektrischen, elektromagnetischen und/oder Ultraschallfeldes, an die magnetischen Nanostrukturen oder durch Einwirkenlassen chemischer und/oder biologischer Reagenzien auf die magnetischen Nanostrukturen beeinflusst wird.

7. Verfahren nach einem der vorhergehenden Ansprüche, wobei sich zumindest ein Teil der magnetischen Nanostrukturen während der Ausführung der Schritte des Verfahrens zum Erfassen des Wachstums von magnetischen Nanostrukturen in der Messkammer (11) befindet und das Wachstum der magnetischen Nanostrukturen außerhalb der Messkammer (11) beeinflusst wird und wobei zumindest ein Teil der magnetischen Nanostrukturen zwischen der Ausführung der Schritte des Verfahrens zum Erfassen des Wachstums von magnetischen Nanostrukturen und dem Schritt der Beeinflussung des Wachstums der magnetischen Nanostrukturen in die Messkammer (11) hinein oder aus dieser heraus gepumpt wird.

8. Verfahren zum Gewinnen von magnetischen Nanostrukturen, wobei das Verfahren zum Steuern des Wachstums von magnetischen Nanostrukturen nach einem der vorhergehenden Ansprüche ausgeführt wird und wobei zusätzlich zumindest ein Teil der magnetischen Nanostrukturen aufgrund ihrer physikalischen und/oder magnetischen Eigenschaften von den übrigen magnetischen Nanostrukturen getrennt wird und getrennt von den übrigen magnetischen Nanostrukturen gespeichert wird.

9. Vorrichtung zur Steuerung des Wachstums von magnetischen Nanostrukturen, welche zur Ausführung eines Verfahrens zum Steuern des Wachstums von magnetischen Nanostrukturen nach einem der Ansprüche 1 bis 7 eingerichtet ist, umfassend
- eine Messkammer (11), welche zur Aufnahme von magnetischen Nanostrukturen eingerichtet ist;
- eine Spulenanordnung (12, 13), welche dazu eingerichtet ist, die magnetischen Nanostrukturen in der Messkammer (11) durch ein Magnetfeld anzuregen und das Magnetfeld in der Messkammer (11) zu erfassen;
- eine Auswerteeinheit (1), welche dazu eingerichtet ist, die magnetischen Nanostrukturen anhand des erfassten Magnetfeldes auszuwerten;
und eine Beeinflussungseinheit (21, 22, 30), welche dazu eingerichtet ist, in Abhängigkeit von der Auswertung der magnetischen Nanostrukturen durch die Auswertungseinheit (1) das Wachstum der magnetischen Nanostrukturen zu beeinflussen.

10. Vorrichtung zur Gewinnung magnetischer Nanostrukturen, welche insbesondere zur Ausführung eines Verfahrens zum Gewinnen von magnetischen Nanostrukturen nach Anspruch 8 eingerichtet ist, umfassend eine Vorrichtung zur Steuerung des Wachstums von magnetischen Nanostrukturen nach Anspruch 9, eine Trenneinheit (19), welche dazu eingerichtet ist, zumindest einen Teil der magnetischen Nanostrukturen von den übrigen magnetischen Nanostrukturen aufgrund ihrer physikalischen und/oder magnetischen Eigenschaften zu trennen, und eine Speichereinheit (20), welche dazu eingerichtet ist, diese magnetischen Nanostrukturen getrennt von den übrigen magnetischen Nanostrukturen zu speichern.

## Revendications

1. Procédé d'orientation de la croissance de nanostructures magnétiques, dans lequel un procédé de détection de la croissance de nanostructures magnétiques est exécuté, où un procédé de détection de nanostructures magnétiques avec les étapes de :
- fourniture de nanostructures magnétiques dans une chambre de mesure (11) ;
- excitation des nanostructures magnétiques dans la chambre de mesure (11) par un champ magnétique généré par un agencement de bobines (12, 13) ;
- détection du champ magnétique dans la chambre de mesure (11) par l'agencement de bobines (12, 13) ;
- évaluation des nanostructures magnétiques par l'utilisation du champ magnétique détecté ;
est effectué au moins à deux moments différents à différents stades d'un processus de croissance des nanostructures magnétiques, et dans lequel, de plus, en fonction de l'évaluation des nanostructures magnétiques à un certain moment, la croissance des nanostructures magnétiques est influencée, fournissant ainsi une boucle de rétroaction pour orienter le processus de nucléation et de croissance des nanostructures magnétiques,
**caractérisé en ce que**
les nanostructures magnétiques résident dans la chambre de mesure (11) tout au long de l'exécution du procédé d'orientation de la croissance de nanostructures magnétiques
ou :
dans lequel au moins une partie des nanostructures magnétiques réside dans la chambre de mesure (11) pendant l'exécution des étapes du procédé de détection de la croissance de nanostructures magnétiques, et la croissance des nanostructures magnétiques est influencée à l'extérieur de la chambre de mesure (11).

2. Procédé selon la revendication 1, **caractérisé en ce que**, dans le procédé de détection de nanostructures magnétiques, l'agencement de bobines (12, 13) comprend une bobine de transmission (13) et une bobine de réception (12) qui est différente de la bobine de transmission (13) ou **en ce que** l'agencement de bobines (12, 13) comprend une bobine qui sert à la fois de bobine de transmission et de bobine de réception.

3. Procédé selon l'une des revendications précédentes, **caractérisé en ce que**, dans le procédé de détection de nanostructures magnétiques, la fréquence d'excitation du champ magnétique généré par l'agencement de bobines (12, 13) pour l'excitation des nanostructures magnétiques est atténuée, en particulier par l'utilisation d'un filtre coupe-bande (8) après la bobine de réception, par l'utilisation de la détection parallèle, par l'utilisation d'une bobine de gradiomètre comme bobine de réception, ou par une combinaison de ces techniques.

4. Procédé selon l'une des revendications précédentes, **caractérisé en ce que**, dans le procédé de détection de nanostructures magnétiques, le signal correspondant au champ magnétique détecté par l'agencement de bobines (12, 13) est amplifié, en particulier par un amplificateur à faible bruit (9).

5. Procédé selon l'une des revendications précédentes, **caractérisé en ce que** le procédé de détection de la croissance de nanostructures magnétiques est réalisé en temps réel, c'est-à-dire pendant la croissance des nanostructures magnétiques.

6. Procédé selon l'une des revendications précédentes, dans lequel la croissance des nanostructures magnétiques est influencée par l'application d'un champ externe, en particulier un champ magnétique, électrique, électromagnétique et/ou ultrasonique, aux nanostructures magnétiques ou par l'application de réactifs chimiques et/ou biologiques aux nanostructures magnétiques.

7. Procédé selon l'une des revendications précédentes, dans lequel au moins une partie des nanostructures magnétiques résident dans la chambre de mesure (11) pendant l'exécution des étapes du procédé de détection de la croissance de nanostructures magnétiques, et la croissance des nanostructures magnétiques est influencée à l'extérieur de la chambre de mesure (11) et dans lequel au moins une partie des nanostructures magnétiques sont pompées dans ou hors de la chambre de mesure (11) entre l'exécution des étapes du procédé de détection de la croissance de nanostructures magnétiques et l'étape d'influence de la croissance des nanostructures magnétiques.

8. Procédé d'obtention de nanostructures magnétiques, dans lequel le procédé d'orientation de la croissance de nanostructures magnétiques selon l'une des revendications précédentes est exécuté et dans lequel, de plus, au moins une partie des nanostructures magnétiques sont séparées des nanostructures magnétiques restantes sur la base de leurs propriétés physiques et/ou magnétiques et sont stockées séparément des nanostructures magnétiques restantes.

9. Dispositif d'orientation de la croissance de nanostructures magnétiques, adapté pour exécuter un procédé d'orientation de la croissance de nanostructures magnétiques selon l'une des revendications 1 à 7, comprenant
- une chambre de mesure (11) adaptée pour accueillir des nanostructures magnétiques,
- un agencement de bobines (12, 13) adapté pour exciter les nanostructures magnétiques dans la chambre de mesure (11) par un champ magnétique et pour détecter le champ magnétique dans la chambre de mesure (11) ;
- une unité d'évaluation (1) adaptée pour évaluer les nanostructures magnétiques par l'utilisation du champ magnétique détecté ;
et une unité d'influence (21, 22, 30) adaptée, en fonction de l'évaluation des nanostructures magnétiques par l'unité d'évaluation (1), pour influencer la croissance des nanostructures magnétiques.

10. Dispositif d'obtention de nanostructures magnétiques, en particulier adapté pour exécuter un procédé d'obtention de nanostructures magnétiques selon la revendication 8, comprenant un dispositif d'orientation de la croissance de nanostructures magnétiques selon la revendication 9, une unité de séparation (19) adaptée pour séparer au moins une partie des nanostructures magnétiques des nanostructures magnétiques restantes sur la base de leurs propriétés physiques et/ou magnétiques, et une unité de stockage (20) adaptée pour stocker ces nanostructures magnétiques séparément des nanostructures magnétiques restantes.
